# EUROPEAN PATENT APPLICATION

(11) **EP 3 929 195 A1**
(43) Date of publication of application: **29.12.2021**
(21) Application number: 20759694.1
(22) Date of filing: 17.02.2020
(51) Int. Cl.: C07D 471/04, A61K 31/519, A61P 35/00

(54) **NOVEL PYRIDO[3,4-D]PYRIMIDIN-8-ONE DERIVATIVE HAVING PROTEIN KINASE INHIBITORY ACTIVITY, AND PHARMACEUTICAL COMPOSITION FOR PREVENTING, ALLEVIATING, OR TREATING CANCER, COMPRISING SAME**

(30) Priority: 18.02.2019 KR 20190018704
(71) Applicant: Korea Institute of Science and Technology, Seoul 02792 (KR)
(72) Inventor: SIM, Tae Bo, Seoul 02792 (KR); HUR, Woo Young, Seoul 02792 (KR); SONG, Chi Man, Seoul 02792 (KR); SENGUPTA, Sandip, Seoul 02792 (KR); PARK, Chan Sun, Seoul 02792 (KR); CHOI, Seung Hye, Seoul 02792 (KR); CHO, Han Na, Seoul 02792 (KR); SHIN, In Jae, Seoul 02792 (KR)
(74) Representative: Weiss, Wolfgang
(86) International application number: PCT/KR2020/002213
(87) International publication number: WO 2020/171499

(57) **Abstract**

The present disclosure relates to a pyrido[3,4-d]pyrimidin-8-one derivative compound exhibiting excellent anti-proliferative effects against cancer cells, a pharmaceutically acceptable salt thereof, a hydrate thereof, or a stereoisomer thereof, a production method therefor, a pharmaceutical composition for preventing, alleviating or treating cancer metastasis and proliferative disease containing the same as an active ingredient, and an anticancer composition against cancer cells. The compound exhibits excellent cancer cell inhibitory activity and anti-proliferative effects, and thus is effective in inhibiting cancer cells, preventing cancer metastasis and proliferative diseases or treating cancer.

## Description

### Technical Field

The present disclosure relates to a compound selected from among a novel pyrido[3,4-d]pyrimidin-8-one derivative having protein kinase inhibitory activity, a pharmaceutically acceptable salt thereof, a hydrate thereof, and a stereoisomer thereof, a method for producing the compound, and a pharmaceutical composition for preventing, alleviating or treating cancer containing the compound.

### Background Art

Protein kinases are enzymes that catalyze phosphorylation reactions that transfer a gamma-phosphate group from ATP to the hydroxyl groups of tyrosine, serine and threonine on a protein. They are involved in cellular metabolism, gene expression, cell growth, differentiation and cell division, and play an important role in cell signaling.

Protein kinases account for about 2% of the eukaryotic genome, and there are about 518 protein kinases in the human genome. Protein kinases are classified into tyrosine protein kinases that phosphorylate tyrosine, and serine/threonine kinases that phosphorylate serine and threonine. Among them, more than about 90 kinases are tyrosine kinases, and are divided into receptor tyrosine kinases (RTKs) and non-receptor tyrosine kinases (NRTKs). Receptor tyrosine kinases are membrane proteins that have domains capable of receiving growth factors on the cell surface, and have active sites capable of phosphorylating tyrosine residues in the cytoplasm. Non-receptor tyrosine kinases are single tyrosine kinase domains present in the nucleus and cytoplasm, and phosphorylate tyrosine residues by receiving signals even though they are not receptors.

Protein kinases are molecular switches and the transition between active and inactive states thereof in cells should be smoothly regulated. When they are abnormally regulated, they excessively activate excessive intracellular signaling, resulting in uncontrollable cell division and proliferation. In addition, abnormal activation of protein kinases by gene mutation, amplification and overexpression is associated with the development and progression of various tumors, and thus plays a decisive role in the growth and metastasis of cancer cells. Typical examples of protein kinases that are abnormally regulated include EGFR, VEGFR, PDGFRB, c-KIT, ABL1, SRC, BRAF, FGFR, BTK, SYK, ALK, MET, CDK, MEK, mTOR, JAK, LCK, PLK, RSK, LYN, FMS, TIE2, RET, AKT, MAP, FAK, DDR, FLT3, and FES. In particular, since receptor tyrosine kinases are mainly involved in external signaling pathways for cell growth and signaling pathways for internal responses, inhibition thereof may also lead to cancer cell growth inhibition and death.

Due to these characteristics, inhibition of kinase activity has attracted attention as a major target for the development of anticancer drugs, and studies on the development of low-molecular-weight organic compounds targeting various kinases have been actively conducted.

Examples of kinase inhibitors include Gleevec^{®} (imatinib, Novartis) which is a Bcr-Abl and PDGFR tyrosine kinase inhibitor, Herceptin^{®} (trastuzumab, Genentech) which is an Her-2 antibody, Iressa^{®} (gefitinib, AstraZeneca) which is an EGFR inhibitor, Nexavar^{®} (sorafenib, Bayer) which is an inhibitor of Raf, VEGFR, KIT, RET, PDGFR-B and FLT-3, Zelboraf^{®} (vemurafenib, Roche) which is a BRAF inhibitor, Erbitux^{®} (cetuximab, Imclone) which is an EGFR antibody, Tarceva^{®} (erlotinib, Genentech/Roche) which is an EGFR inhibitor, and Sutent^{®} (sunitinib, Pfizer) which is a KDR inhibitor. They have been approved by the FDA for use as anticancer drugs for leukemia, breast cancer, non-small cell lung cancer, liver cancer, malignant melanoma, colorectal cancer and the like, and are widely used as first-line standard therapy due to their excellent therapeutic efficacy. In addition, various compounds are in clinical trials.

Meanwhile, acute myeloid leukemia (AML) is one of fatal blood diseases in which blood cells differentiate abnormally and proliferate continuously. More than 16% of acute myeloid leukemia (AML) patients have a point-mutated RAS (small G protein) protein, and NRAS mutations account for the majority (10% or more) of RAS kinases. For this reason, NRAS G protein has been considered as a promising drug target for the treatment of AML. When the protooncogene RAS is mutated, RAS is continuously activated (gain-of-function), and various signaling pathways downstream of RAS are activated to accelerate cancer cell growth.

Over the last 40 years, RAS point mutations or key signaling molecules downstream of RAS have been proposed as targets. However, they did not lead to *in vivo* experiments and clinical trials due to the complexity and compensatory effect of mutant RAS signaling pathways. For example, selumetinib (AZD 6244), which inhibits MEK, a key molecule downstream of RAS, showed no therapeutic effect in all three AML patients with a NRAS mutant gene in phase 2 clinical trials. In addition, in an attempt to find targets, RNA interference screening was used to identify proteins (TBK1, STK33 and GATA2) which are genetically in a synthetic lethal relationship with KRAS mutation. However, this also failed to achieve clinical therapeutic effects. In particular, in the case of STK33, it was proven through cell-based pharmacological screening at the preclinical stage that therapeutic strategies using KRAS mutation and synthetic lethal principles cannot be established. In addition, in recent years, cell-based pharmacological screening was used to identify a compound (GNF 7) that selectively inhibits the RAS mutant signaling pathway, and the inhibitory effects thereof in a preclinical leukemia model were confirmed. The mechanism of action of the GNF 7 compound is to simultaneously inhibit two kinases, GCK and ACK1, which specifically contribute to cell growth downstream of the RAS mutant signaling pathway. This compound also actually exhibited its efficacy in cell samples from AML patients with NRAS mutants. Inhibitors against two kinases, GCK and ACK1, are known to be effective for the treatment of cancer diseases caused by NRAS mutation, such as melanoma, colorectal cancer, thyroid cancer, and various blood cancers.

### [Prior Art Documents]

### [Patent Documents]

### (Patent Document 1) International Patent Publication No. WO 2015-011597

### [Non-Patent Documents]

(Non-Patent Document 1) Choi HG, Ren P, Adrian F, et al. A type-II kinase inhibitor capable of inhibiting the T315I "gatekeeper" mutant of Bcr-Abl. J. Med. Chem., 2010; 53(15): 5439-5448.

(Non-Patent Document 2) Nonami, A.; Sattler, M.; Weisberg, E.; Liu, Q.; Zhang, J.; Patricelli, M. P.; Christie, A. L.; Saur, A. M.; Kohl, N. E.; Kung, A. L.; Yoon, H.; Sim, T.; Gray, N. S.; Griffin, J. D., Identification of novel therapeutic targets in acute leukemias with NRAS mutations using a pharmacologic approach. Blood 2015, 125 (20), 3133-43.

(Non-Patent Document 3) Luo T, Masson K, Jaffe JD, et al. STK33 kinase inhibitor BRD-8899 has no effect on KRASdependent cancer cell viability. Proc Natl Acad Sci USA. 2012; 109(8): 2860-2865.

(Non-Patent Document 4) Jain N, Curran E, Iyengar NM, et al. Phase II study of the oral MEK inhibitor selumetinib in advanced acute myelogenous leukemia: a University of Chicago phase II consortium trial. Clin Cancer Res. 2014; 20(2): 490-498.

(Non-Patent Document 5) Johnson, D. B.; Smalley, K. S.; Sosman, J. A., Molecular pathways: targeting NRAS in melanoma and acute myelogenous leukemia. Clin Cancer Res 2014, 20(16), 4186-92.

(Non-Patent Document 6) H Cho, I Shin, E Ju, et al. First SAR Study for Overriding NRAS Mutant Driven Acute Myeloid Leukemia. J. Med. Chem. 2018, 61 (18), 8353-8373.

### DISCLOSURE

### Technical Problem

Therefore, an object of the present disclosure is to provide a novel pyrido[3,4-d]pyrimidin-8-one derivative compound having protein kinase inhibitory activity.

Another object of the present disclosure is to provide a pharmaceutical composition useful for the treatment, prevention and alleviation of cancer disease, the pharmaceutical composition containing, as an active ingredient, a novel pyrido[3,4-d]pyrimidin-8-one derivative compound, a pharmaceutically acceptable salt thereof, a hydrate thereof, a solvate thereof, or a stereoisomer thereof.

Still another object of the present disclosure is to provide a therapeutic agent for a cancer disease caused by NRAS mutation, the therapeutic agent containing, as an active ingredient, a novel pyrido[3,4-d]pyrimidin-8-one derivative compound, a pharmaceutically acceptable salt thereof, a hydrate thereof, a solvate thereof, or a stereoisomer thereof.

### Technical Solution

In order to achieve the above objects, the present disclosure provides a compound selected from among a pyrido[3,4-d]pyrimidin-8-one derivative compound represented by the following Formula 1, a pharmaceutically acceptable salt thereof, a hydrate thereof, and a stereoisomer thereof: 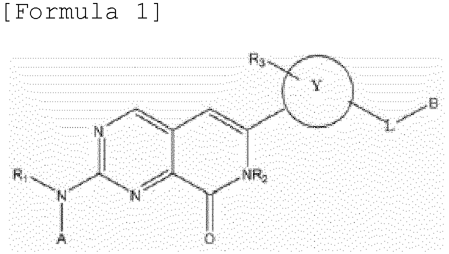 wherein
B is hydrogen, a C₁-C₁₃ alkyl group, a C₆-C₁₀ aryl group, a C₃-C₁₀ cyclyl group, a C₃-C₁₀ heteroaryl group, a C₃-C₁₀ heterocyclyl group, or -C (O) - (C₁-C₁₃ alkyl);
A is hydrogen, a C₁-C₁₃ alkyl group, a C₆-C₁₀ aryl group, a C₃-C₁₀ cyclyl group, a C₃-C₁₀ heteroaryl group, a C₃-C₁₀ heterocyclyl group, or -C (O) - (C₁-C₁₃ alkyl), or A together with a nitrogen atom to which R₁ is attached forms a 4- to 7-membered saturated, unsaturated or aromatic ring, which may optionally contain at least one of N, O, S, NH, C=N, C=O, -NHC(O)-, -NHC(O)NH-, -NHS(O)₂- and SO₂ and may optionally be substituted with at least one of a C₁-C₁₃ alkyl group, a C₆-C₁₀ aryl group, a C₃-C₁₀ heteroaryl group, a hydroxyl group, a halide group and a cyano group;
R₁ is hydrogen, a C₁-C₁₃ alkyl group, a C₃-C₁₀ cyclyl group, or a C₃-C₁₀ heterocyclyl group, or R₁ together with a nitrogen atom to which A is attached forms a 4- to 7-membered saturated, unsaturated or aromatic ring, which may optionally contain at least one of N, O, S, NH, C=N, C=O, - NHC(O)-, -NHC(O)NH-, -NHS(O)₂- and SO₂ and may optionally be substituted with at least one of a C₁-C₁₃ alkyl group, a C₆-C₁₀ aryl group, a C₃-C₁₀ heteroaryl group, a hydroxyl group, a halide group and a cyano group;
R₂ and R₃ are each hydrogen, a hydroxyl group, a halogen group, a C₁-C₆ alkyl group, a C₁-C₆ alkenyl group, a C₆-C₁₀ aryl group, a C₃-C₁₀ heteroaryl group, or a C₃-C₁₀ heterocyclyl group;
Y is a C₆-C₁₀ aryl group, or a 5- to 9-membered heteroaryl group containing 1 to 4 heteroatoms selected from among nitrogen (N), oxygen (O) and sulfur (S) atoms;
L is selected from the group consisting of -NR₄-, - NR₄CH₂-, -NR₄C(O)-, -C(O)NR₄-, -NR₄C(O)NR₄-, -S(O)₂-, - NR₄S(O)₂-, and -S(O)₂NR₄-;
R₄ is hydrogen or a C₁-C₆ alkyl group;
the C₁-C₆ alkyl group, the C₁-C₁₃ alkyl group or the C₃-C₁₀ cyclyl group contains at least one substituent selected from the group consisting of hydrogen, a hydroxyl group, a halogen group, a C₁-C₁₃ alkyl group, a C₁-C₆ alkoxy group, an amino group (-NR₅R₆), a nitro group (-N(O)₂), an amide group (-(C=O)NR₅R₆), a carboxylic acid group (-C(O)OH), a nitrile group (-CN), a urea group (-NR₅(C=O)NR₆-), a sulfonamide group (-NHS(O)₂-), a sulfide group (-S-), a sulfone group (-S(O)₂-), a phosphiryl group (-P(O)R₅R₆), a C₆-C₁₀ aryl group, a C₃-C₁₀ heteroaryl group, and a C₃-C₁₀ heterocyclyl group;
the C₆-C₁₀ aryl group, the C₃-C₁₀ heteroaryl group or the C₃-C₁₀ heterocyclyl group contains at least one substituent selected from the group consisting of hydrogen, a hydroxyl group, a halogen group, a carbonyl group (-(C=O)R₅R₆), a C₁-C₃ alkyl group unsubstituted or substituted with a halogen or C₃-C₁₀ heterocyclyl group, a C₁-C₃ alkoxy group unsubstituted or substituted with a halogen or C₃-C₁₀ heterocyclyl group, C₆-C₁₀ phenoxy, an amino group (-NR₅R₆), a nitro group (-N(O)₂), an amide group (-(C=O)NR₅R₆), a carboxylic acid group (-C(O)OH), a nitrile group (-CN), a urea group (-NR₅(C=O)NR₆-), a sulfonamide group (-NHS(O)₂-), a sulfide group (-S-), a sulfone group (-S(O)₂-), a phosphiryl group (-P(O)R₅R₆), a C₆-C₁₀ aryl group, a C₃-C₁₀ heteroaryl group, and a C₃-C₁₀ heterocyclyl group;
R₅ and R₆ contain at least one selected from the group consisting of hydrogen, a C₁-C₆ alkyl group, a C₁-C₆ alkenyl group, a C₁-C₆ alkynyl group, a C₆-C₁₀ aryl group, a C₃-C₁₀ heteroaryl group, and a C₃-C₁₀ heterocyclyl group; and
the C₃-C₁₀ heteroaryl group and the C₃-C₁₀ heterocyclyl group contain at least one heteroatom selected from the group consisting of N, O and S.

### Advantageous Effects

The compound according to the present disclosure has excellent ability to inhibit the activity of protein kinase such as ABL1, FGFR2, TAOK2/TAO1, EPHA5, EPHB2, EPHB3, RET, LYN B, EPHA2, FRK/PTK5, EPHA8, LCK, EPHB4, FYN, KHS/MAP4K5, DDR1, EPHA3, P38a/MAPK14, EPHA4, FMS, EPHB1, HCK, FGFR1, ABL2/ARG, EPHA6, c-Src, ACK1, FLT4/VEGFR3, ERBB4/HER4, DDR2, KDR/VEGFR2, LYN, ZAK/MLTK, YES/YES1, BLK, FGR, MLCK2/MYLK2, TAOK1, BMX/ETK, BTK, EPHA1, JAK1, P38b/MAPK11, TIE2/TEK, FLT1/VEGFR1, TXK, SRMS, RAF1, SIK1, MLK3/MAP3K11, PEAK1, TRKA, EPHA7, GLK/MAP4K3, MLK2/MAP3K10, TEC, CSK, TRKC, FES/FPS, SIK2, FGFR3, BRK, YSK4/MAP3K19, ARAF, PDGFRb, TNK1, GCK/MAP4K2, PDGFRa, TNIK, TAK1, ERBB2/HER2, LIMK1, HIPK4, FER, EGFR, JAK2, HPK1/MAP4K1, TRKB, RIPK3, LOK/STK10, LIMK2, MLK1/MAP3K9, BRAF, MEKK3, MEK5, STK32B/YANK2, FGFR4, MEKK2, SLK/STK2, FLT3, PKAcg, TAOK3/JIK, TYRO3/SKY, SIK3, IR, LRRK2, PYK2, NEK11, p70S6K/RPS6KB1, or LATS2. Thus, the compound may be used for the purpose of treating, preventing and alleviating cancer disease caused by abnormal cell growth.

The compound according to the present disclosure, a pharmaceutically acceptable salt thereof, or a hydrate thereof, and a pharmaceutical composition for preventing or treating cancer containing the same as an active ingredient exhibits high inhibitory activity and an antiproliferative effect selectively against cancer cells while showing low cytotoxicity, and thus may be effectively used for the prevention or treatment of cancer.

Cancer diseases that may be treated, prevented and alleviated by treatment with the compound according to the present disclosure include stomach cancer, lung cancer, liver cancer, colorectal cancer, small intestine cancer, pancreatic cancer, brain cancer, bone cancer, melanoma, breast cancer, sclerosing adenosis, uterine cancer, cervical cancer, head and neck cancer, esophageal cancer, thyroid cancer, parathyroid cancer, kidney cancer, sarcoma, prostate cancer, urethral cancer, bladder cancer, blood cancer (including leukemia, multiple myeloma, and myelodysplastic syndrome), lymphoma (Hodgkin's disease, and non-Hodgkin's lymphoma), psoriasis, or fibroadenoma.

In particular, the compound according to the present disclosure has excellent inhibitory activity against two kinases, GCK and ACK1, and thus is effective for the treatment of cancer disease caused by NRAS mutation, such as melanoma, colorectal cancer, thyroid cancer, or acute myeloid leukemia (AML).

### Best Mode

Since all numbers, values and/or expressions referring to quantities of components, reaction conditions, and mixtures used in the present specification are subject to various uncertainties of measurement encountered in obtaining such values, unless otherwise indicated, all are to be understood as modified in all instances by the term "about." Where a numerical range is disclosed herein, such a range is continuous, inclusive of both the minimum and maximum values of the range as well as every value between such minimum and maximum values, unless otherwise indicated. Still further, where such a range refers to integers, every integer between the minimum and maximum values of such a range is included, unless otherwise indicated.

In the present specification, where a range is stated for a parameter, it will be understood that the parameter includes all values within the stated range, inclusive of the stated endpoints of the range. For example, a range of 5 to 10 will be understood to include the values 5, 6, 7, 8, 9, and 10, as well as any sub-range such as 6 to 10, 7 to 10, 6 to 9, and 7 to 9, and also include any value and range between the integers which are reasonable in the context of the range stated, such as 5.5, 6.5, 7.5, 5.5 to 8.5 and 6.5 to 9. For example, a range of "10% to 30%" will be understood to include the values 10%, 11%, 12%, 13%, etc., and all integers up to and including 30%, as well as any sub-range such as 10% to 15%, 12% to 18%, 20% to 30%, etc., and also include any value between the integers which are reasonable in the context of the range stated, such as 10.5%, 15.5%, 25.5%, etc.

Hereinafter, the present disclosure will be described in detail.

The present inventors have conducted extensive studies to solve the above-described problems, and as a result, have developed an anticancer compound exhibiting excellent inhibitory activity against cancer cells, particularly a pyrido[3,4-d]pyrimidin-8-one derivative compound useful for the prevention or treatment of cancer as a selective kinase activity inhibitor, a pharmaceutically acceptable salt thereof, a hydrate thereof, and a stereoisomer thereof, or a method for producing the same, and a pharmaceutical composition for preventing or treating cancer containing the same as an active ingredient.

One aspect of the present disclosure provides a compound selected from among a pyrido[3,4-d]pyrimidin-8-one derivative compound represented by the following Formula 1, a pharmaceutically acceptable salt thereof, a hydrate thereof, and a stereoisomer thereof: 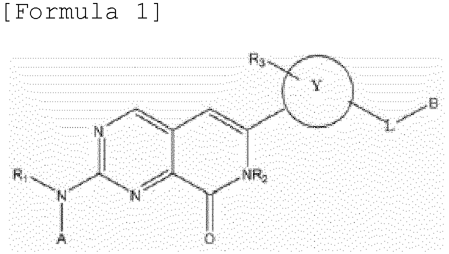
wherein
B is hydrogen, a C₁-C₁₃ alkyl group, a C₆-C₁₀ aryl group, a C₃-C₁₀ cyclyl group, a C₃-C₁₀ heteroaryl group, a C₃-C₁₀ heterocyclyl group, or -C(O)-(C₁-C₁₃ alkyl);
A is hydrogen, a C₁-C₁₃ alkyl group, a C₆-C₁₀ aryl group, a C₃-C₁₀ cyclyl group, a C₃-C₁₀ heteroaryl group, a C₃-C₁₀ heterocyclyl group, or -C(O)-(C₁-C₁₃ alkyl), or A together with a nitrogen atom to which R₁ is attached forms a 4- to 7-membered saturated, unsaturated or aromatic ring, which may optionally contain at least one of N, O, S, NH, C=N, C=O, -NHC(O)-, -NHC(O)NH-, -NHS(O)₂- and SO₂ and may optionally be substituted with at least one of a C₁-C₁₃ alkyl group, a C₆-C₁₀ aryl group, a C₃-C₁₀ heteroaryl group, a hydroxyl group, a halide group and a cyano group;
R₁ is hydrogen, a C₁-C₁₃ alkyl group, a C₃-C₁₀ cyclyl group, or a C₃-C₁₀ heterocyclyl group, or R₁ together with a nitrogen atom to which A is attached forms a 4- to 7-membered saturated, unsaturated or aromatic ring, which may optionally contain at least one of N, O, S, NH, C=N, C=O, - NHC(O)-, -NHC(O)NH-, -NHS(O)₂- and SO₂ and may optionally be substituted with at least one of a C₁-C₁₃ alkyl group, a C₆-C₁₀ aryl group, a C₃-C₁₀ heteroaryl group, a hydroxyl group, a halide group and a cyano group;
R₂ and R₃ are each hydrogen, a hydroxyl group, a halogen group, a C₁-C₆ alkyl group, a C₁-C₆ alkenyl group, a C₆-C₁₀ aryl group, a C₃-C₁₀ heteroaryl group, or a C₃-C₁₀ heterocyclyl group;
Y is a C₆-C₁₀ aryl group, or a 5- to 9-membered heteroaryl group containing 1 to 4 heteroatoms selected from among nitrogen (N), oxygen (O) and sulfur (S) atoms;
L is selected from the group consisting of -NR₄-, - NR₄CH₂-, -NR₄C(O)-, -C(O)NR₄-, -NR₄C(O)NR₄-, -S(O)₂-, - NR₄S(O)₂-, and -S(O)₂NR₄-;
R₄ is hydrogen or a C₁-C₆ alkyl group;
the C₁-C₆ alkyl group, the C₁-C₁₃ alkyl group or the C₃-C₁₀ cyclyl group contains at least one substituent selected from the group consisting of hydrogen, a hydroxyl group, a halogen group, a C₁-C₁₃ alkyl group, a C₁-C₆ alkoxy group, an amino group (-NR₅R₆), a nitro group (-N(O)₂), an amide group (-(C=O)NR₅R₆), a carboxylic acid group (-C(O)OH), a nitrile group (-CN), a urea group (-NR₅(C=O)NR₆-), a sulfonamide group (-NHS(O)₂-), a sulfide group (-S-), a sulfone group (-S(O)₂-), a phosphiryl group (-P(O)R₅R₆), a C₆-C₁₀ aryl group, a C₃-C₁₀ heteroaryl group, and a C₃-C₁₀ heterocyclyl group;
the C₆-C₁₀ aryl group, the C₃-C₁₀ heteroaryl group or the C₃-C₁₀ heterocyclyl group contains at least one substituent selected from the group consisting of hydrogen, a hydroxyl group, a halogen group, a carbonyl group (-(C=O)R₅R₆), a C₁-C₃ alkyl group unsubstituted or substituted with a halogen or C₃-C₁₀ heterocyclyl group, a C₁-C₃ alkoxy group unsubstituted or substituted with a halogen or C₃-C₁₀ heterocyclyl group, C₆-C₁₀ phenoxy, an amino group (-NR₅R₆), a nitro group (-N(O)₂), an amide group (-(C=O)NR₅R₆), a carboxylic acid group (-C(O)OH), a nitrile group (-CN), a urea group (-NR₅(C=O)NR₆-), a sulfonamide group (-NHS(O)₂-), a sulfide group (-S-), a sulfone group (-S(O)₂-), a phosphiryl group (-P(O)R₅R₆), a C₆-C₁₀ aryl group, a C₃-C₁₀ heteroaryl group, and a C₃-C₁₀ heterocyclyl group;
R₅ and R₆ contain at least one selected from the group consisting of hydrogen, a C₁-C₆ alkyl group, a C₁-C₆ alkenyl group, a C₁-C₆ alkynyl group, a C₆-C₁₀ aryl group, a C₃-C₁₀ heteroaryl group, and a C₃-C₁₀ heterocyclyl group; and
the C₃-C₁₀ heteroaryl group and the C₃-C₁₀ heterocyclyl group contain at least one heteroatom selected from the group consisting of N, O and S.

In one aspect of the present disclosure, there is provided a compound selected from the pyrido[3,4-d]pyrimidin-8-one derivative compound represented by Formula 1, a pharmaceutically acceptable salt thereof, a hydrate thereof, and a stereoisomer thereof, wherein the pyrido[3,4-d]pyrimidin-8-one derivative compound represented by Formula 1 is any one of compounds represented by the following Formulas 2 to 9: 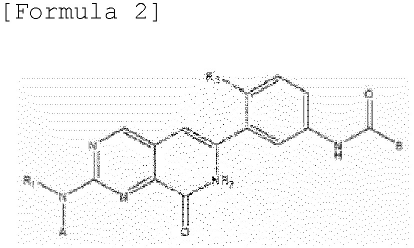 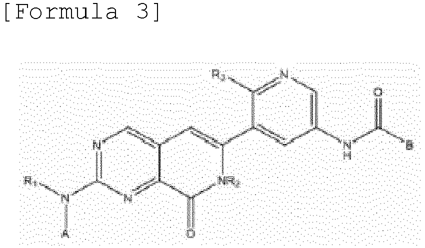 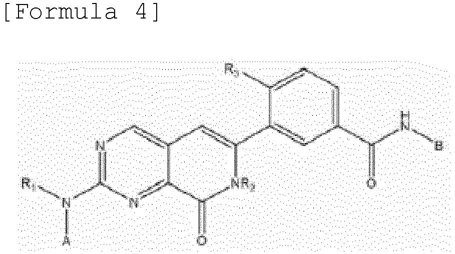 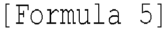 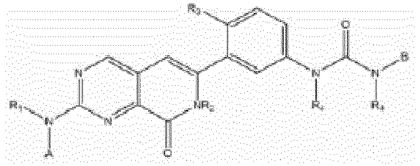 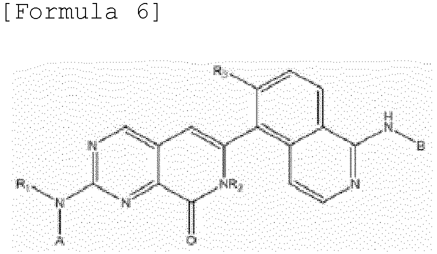 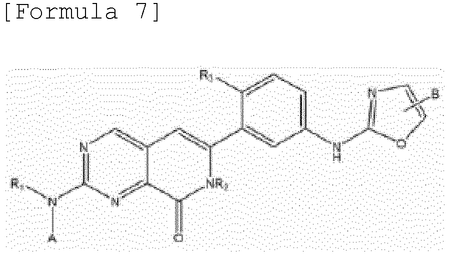 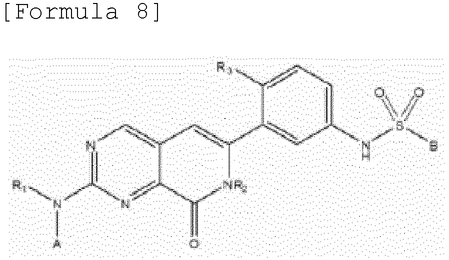 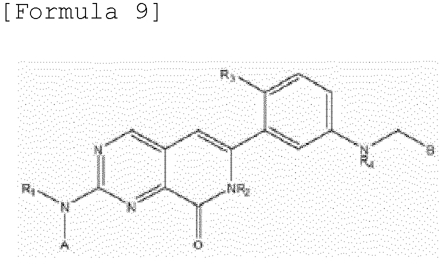 wherein
B is hydrogen, a C₁-C₁₃ alkyl group, a C₆-C₁₀ aryl group, a C₃-C₁₀ cyclyl group, a C₃-C₁₀ heteroaryl group, a C₃-C₁₀ heterocyclyl group, or -C(O)-(C₁-C₁₃ alkyl);
A is hydrogen, a C₁-C₁₃ alkyl group, a C₆-C₁₀ aryl group, a C₃-C₁₀ cyclyl group, a C₃-C₁₀ heteroaryl group, a C₃-C₁₀ heterocyclyl group, or -C(O)-(C₁-C₁₃ alkyl), or A together with a nitrogen atom to which R₁ is attached forms a 4- to 7-membered saturated, unsaturated or aromatic ring, which may optionally contain at least one of N, O, S, NH, C=N, C=O, -NHC(O)-, -NHC(O)NH-, -NHS(O)₂- and SO₂ and may optionally be substituted with at least one of a C₁-C₁₃ alkyl group, a C₆-C₁₀ aryl group, a C₃-C₁₀ heteroaryl group, a hydroxyl group, a halide group and a cyano group;
R₁ is hydrogen, a C₁-C₁₃ alkyl group, a C₃-C₁₀ cyclyl group, or a C₃-C₁₀ heterocyclyl group, or R₁ together with a nitrogen atom to which A is attached forms a 4- to 7-membered saturated, unsaturated or aromatic ring, which may optionally contain at least one of N, O, S, NH, C=N, C=O, - NHC(O)-, -NHC(O)NH-, -NHS(O)₂- and SO₂ and may optionally be substituted with at least one of a C₁-C₁₃ alkyl group, a C₆-C₁₀ aryl group, a C₃-C₁₀ heteroaryl group, a hydroxyl group, a halide group and a cyano group;
R₂ and R₃ are each hydrogen, a hydroxyl group, a halogen group, a C₁-C₆ alkyl group, a C₁-C₆ alkenyl group, a C₆-C₁₀ aryl group, a C₃-C₁₀ heteroaryl group, or a C₃-C₁₀ heterocyclyl group;
R₄ is hydrogen or a C₁-C₆ alkyl group;
the C₁-C₆ alkyl group, the C₁-C₁₃ alkyl group or the C₃-C₁₀ cyclyl group contains at least one substituent selected from the group consisting of hydrogen, a hydroxyl group, a halogen group, a C₁-C₁₃ alkyl group, a C₁-C₆ alkoxy group, an amino group (-NR₅R₆), a nitro group (-N(O)₂), an amide group (-(C=O)NR₅R₆), a carboxylic acid group (-C(O)OH), a nitrile group (-CN), a urea group (-NR₅(C=O)NR₆-), a sulfonamide group (-NHS(O)₂-), a sulfide group (-S-), a sulfone group (-S(O)₂-), a phosphiryl group (-P(O)R₅R₆), a C₆-C₁₀ aryl group, a C₃-C₁₀ heteroaryl group, and a C₃-C₁₀ heterocyclyl group;
the C₆-C₁₀ aryl group, the C₃-C₁₀ heteroaryl group or the C₃-C₁₀ heterocyclyl group contains at least one substituent selected from the group consisting of hydrogen, a hydroxyl group, a halogen group, a carbonyl group (-(C=O)R₅R₆), a C₁-C₃ alkyl group unsubstituted or substituted with a halogen or C₃-C₁₀ heterocyclyl group, a C₁-C₃ alkoxy group unsubstituted or substituted with a halogen or C₃-C₁₀ heterocyclyl group, C₆-C₁₀ phenoxy, an amino group (-NR₅R₆), a nitro group (-N(O)₂), an amide group (-(C=O)NR₅R₆), a carboxylic acid group (-C(O)OH), a nitrile group (-CN), a urea group (-NR₅(C=O)NR₆-), a sulfonamide group (-NHS(O)₂-), a sulfide group (-S-), a sulfone group (-S(O)₂-), a phosphiryl group (-P(O)R₅R₆), a C₆-C₁₀ aryl group, a C₃-C₁₀ heteroaryl group, and a C₃-C₁₀ heterocyclyl group;
R₅ and R₆ contain at least one selected from the group consisting of hydrogen, a C₁-C₆ alkyl group, a C₁-C₆ alkenyl group, a C₁-C₆ alkynyl group, a C₆-C₁₀ aryl group, a C₃-C₁₀ heteroaryl group, and a C₃-C₁₀ heterocyclyl group; and
the C₃-C₁₀ heteroaryl group and the C₃-C₁₀ heterocyclyl group contain at least one heteroatom selected from the group consisting of N, O and S.

In one aspect of the present disclosure, there is provided a compound selected from among the pyrido[3,4-d]pyrimidin-8-one derivative compound represented by Formula 1, a pharmaceutically acceptable salt thereof, a hydrate thereof, and a stereoisomer thereof, wherein B is hydrogen, a C₁-C₁₃ alkyl group, a C₆-C₁₀ aryl group, a C₃-C₁₀ cyclyl group, or a C₃-C₁₀ heteroaryl group;
A is hydrogen, a C₁-C₁₃ alkyl group, a C₆-C₁₀ aryl group, a C₃-C₁₀ cyclyl group, or a C₃-C₁₀ heteroaryl group, or A together with a nitrogen atom to which R₁ is attached forms a 4- to 7-membered saturated or unsaturated ring, which contains at least one of N, O, S, NH, C=N, C=O, - NHC(O)-, -NHC(O)NH-, -NHS(O)₂- and SO₂ and may optionally be substituted with at least one of a C₁-C₁₃ alkyl group, a C₆-C₁₀ aryl group, a C₃-C₁₀ heteroaryl group, a hydroxyl group, a halide group and a cyano group;
R₁ is hydrogen or a C₁-C₁₃ alkyl group, or R₁ together with a nitrogen atom to which A is attached forms a 4- to 7-membered saturated or unsaturated ring, which contains at least one of N, O, S, NH, C=N, C=O, -NHC(O)-, -NHC(O)NH-, - NHS(O)₂- and SO₂ and may optionally be substituted with at least one of a C₁-C₁₃ alkyl group, a C₆-C₁₀ aryl group, a C₃-C₁₀ heteroaryl group, a hydroxyl group, a halide group and a cyano group;
R₂ and R₃ are each hydrogen, a halogen group, a C₁-C₆ alkyl group, or a C₁-C₆ alkenyl group;
the C₁-C₆ alkyl group, the C₁-C₁₃ alkyl group or the C₃-C₁₀ cyclyl group contains at least one substituent selected from the group consisting of hydrogen, a hydroxyl group, a halogen group, a C₁-C₁₃ alkyl group, a C₁-C₆ alkoxy group, an amide group (-(C=O)NR₅R₆), a C₆-C₁₀ aryl group, a C₃-C₁₀ heteroaryl group, and a C₃-C₁₀ heterocyclyl group;
the C₁-C₆ alkyl group, the C₁-C₁₃ alkyl group or the C₃-C₁₀ cyclyl group contains at least one substituent selected from the group consisting of hydrogen, a hydroxyl group, a halogen group, a C₁-C₁₃ alkyl group, a C₁-C₆ alkoxy group, an amino group (-NR₅R₆), a nitro group (-N(O)₂), an amide group (-(C=O)NR₅R₆), a carboxylic acid group (-C(O)OH), a nitrile group (-CN), a urea group (-NR₅(C=O)NR₆-), a sulfonamide group (-NHS(O)₂-), a sulfide group (-S-), a sulfone group (-S(O)₂-), a phosphiryl group (-P(O)R₅R₆), a C₆-C₁₀ aryl group, a C₃-C₁₀ heteroaryl group, and a C₃-C₁₀ heterocyclyl group;
the C₆-C₁₀ aryl group, the C₃-C₁₀ heteroaryl group or the C₃-C₁₀ heterocyclyl group contains at least one substituent selected from the group consisting of hydrogen, a hydroxyl group, a halogen group, a carbonyl group (-(C=O)R₅R₆), a C₁-C₃ alkyl group unsubstituted or substituted with a halogen or C₃-C₁₀ heterocyclyl group, a C₁-C₃ alkoxy group unsubstituted or substituted with a halogen or C₃-C₁₀ heterocyclyl group, C₆-C₁₀ phenoxy, an amino group (-NR₅R₆), a nitro group (-N(O)₂), an amide group (-(C=O)NR₅R₆), a carboxylic acid group (-C(O)OH), a nitrile group (-CN), a urea group (-NR₅(C=O)NR₆-), a sulfonamide group (-NHS(O)₂-), a sulfide group (-S-), a sulfone group (-S(O)₂-), a phosphiryl group (-P(O)R₅R₆), a C₆-C₁₀ aryl group, a C₃-C₁₀ heteroaryl group, and a C₃-C₁₀ heterocyclyl group;
R₅ and R₆ contain at least one selected from the group consisting of hydrogen, a C₁-C₆ alkyl group, a C₁-C₆ alkenyl group, a C₁-C₆ alkynyl group, a C₆-C₁₀ aryl group, a C₃-C₁₀ heteroaryl group, and a C₃-C₁₀ heterocyclyl group; and
the C₃-C₁₀ heteroaryl group and the C₃-C₁₀ heterocyclyl group contain at least one heteroatom selected from the group consisting of N, O and S.

In one aspect of the present disclosure, there is provided a compound selected from among the pyrido[3,4-d]pyrimidin-8-one derivative compound represented by Formula 1, a pharmaceutically acceptable salt thereof, a hydrate thereof, and a stereoisomer thereof, wherein B is a C₆-C₁₀ aryl group, a C₃-C₁₀ cyclyl group, or a C₃-C₁₀ heteroaryl group;
A is hydrogen, a C₁-C₁₃ alkyl group, a C₆-C₁₀ aryl group, a C₃-C₁₀ cyclyl group, or a C₃-C₁₀ heteroaryl group, or A together with a nitrogen atom to which R₁ is attached forms a 4- to 7-membered saturated or unsaturated ring containing at least one of N, O, NH, C=N, C=O, or SO₂;
R₁ is hydrogen, or R₁ together with a nitrogen atom to which A is attached forms a 4- to 7-membered saturated or unsaturated ring containing at least one of N, O, NH, C=N, C=O and SO₂;
R₂ is hydrogen, a halogen group, a C₁-C₆ alkyl group, or a C₁-C₆ alkenyl group;
R₃ is a C₁-C₆ alkyl group;
R₄ is hydrogen or a C₁-C₆ alkyl group;
the C₁-C₆ alkyl group, the C₁-C₁₃ alkyl group or the C₃-C₁₀ cyclyl group contains at least one substituent selected from the group consisting of hydrogen, a hydroxyl group, a halogen group, a C₁-C₁₃ alkyl group, a C₁-C₆ alkoxy group, an amide group (-(C=O)NR₅R₆), a C₆-C₁₀ aryl group, a C₃-C₁₀ heteroaryl group, and a C₃-C₁₀ heterocyclyl group;
the C₆-C₁₀ aryl group, the C₃-C₁₀ heteroaryl group or the C₃-C₁₀ heterocyclyl group contains at least one substituent selected from the group consisting of hydrogen, a hydroxyl group, a halogen group, a carbonyl group (-(C=O)R₅R₆), a C₁-C₃ alkyl group substituted with a halogen or C₃-C₁₀ heterocyclyl group, a C1-C₃ alkoxy group substituted with a halogen or C₃-C₁₀ heterocyclyl group, C₆-C₁₀ phenoxy, an amino group (-NR₅R₆), an amide group (-(C=O)NR₅R₆), a C₆-C₁₀ aryl group, a C₃-C₁₀ heteroaryl group, and a C₃-C₁₀ heterocyclyl group;
R₅ and R₆ contain at least one selected from the group consisting of hydrogen, a C₁-C₆ alkyl group, a C₁-C₆ alkenyl group, a C₁-C₆ alkynyl group, a C₆-C₁₀ aryl group, a C₃-C₁₀ heteroaryl group, and a C₃-C₁₀ heterocyclyl group; and
the C₃-C₁₀ heteroaryl group and the C₃-C₁₀ heterocyclyl group contain at least one heteroatom selected from the group consisting of N, O and S.

In one aspect of the present disclosure, there is provided a compound selected from among the pyrido[3,4-d]pyrimidin-8-one derivative compound represented by Formula 1, a pharmaceutically acceptable salt thereof, a hydrate thereof, and a stereoisomer thereof, wherein B is a C₁-C₆ alkyl group, substituted or unsubstituted phenyl, substituted or unsubstituted hexane, substituted or unsubstituted furan, substituted or unsubstituted thiophene, substituted or unsubstituted pyridine, substituted or unsubstituted benzofuran, substituted or unsubstituted benzene, substituted or unsubstituted naphthalene, substituted or unsubstituted anthracene, or substituted or unsubstituted phenanthrene; A is hydrogen, substituted or unsubstituted pyridazine, substituted or unsubstituted pyrazine; substituted or unsubstituted imidazole, substituted or unsubstituted pyrazole, substituted or unsubstituted furan, substituted or unsubstituted pyrimidine, substituted or unsubstituted pyrrole, substituted or unsubstituted pyridine, substituted or unsubstituted cyclopropane, substituted or unsubstituted cyclobutane, substituted or unsubstituted ethane, substituted or unsubstituted butane, or substituted or unsubstituted pentane, or A together with a nitrogen atom to which R₁ is attached forms a substituted or unsubstituted morpholino group; R₁ is hydrogen, or R₁ together with a nitrogen atom to which A is attached forms a substituted or unsubstituted morpholino group; R₂ is hydrogen or a substituted or unsubstituted C₁-C₃ alkyl group; R₃ is hydrogen or a C₁-C₃ alkyl group; and R₄ is hydrogen or a C₁-C₆ alkyl group.

In one aspect of the present disclosure, there is provided a compound selected from among the pyrido[3,4-d]pyrimidin-8-one derivative compound represented by Formula 1, a pharmaceutically acceptable salt thereof, a hydrate thereof, and a stereoisomer thereof, wherein the compound is selected from the group consisting of the following Compound Nos. 1 to 55:
(Compound No. 1): N-(3-(2-(cyclopropylamino)-7-methyl-8-oxo-7,8-dihydropyrido[3,4-d]pyrimidin-6-yl)-4-methylphenyl)-3-(trifluoromethyl)benzamide;
(Compound No. 2): N-(3-(2-((2-hydroxyethyl)amino)-7-methyl-8-oxo-7,8-dihydropyrido[3,4-d]pyrimidin-6-yl)-4-methylphenyl)-3-(trifluoromethyl)benzamide;
(Compound No. 3): N-(4-methyl-3-(7-methyl-2-(methylamino)-8-oxo-7,8-dihydropyrido[3,4-d]pyrimidin-6-yl)phenyl)-3-(trifluoromethyl)benzamide;
(Compound No. 4): N-(4-methyl-3-(7-methyl-2-(oxetan-3-ylamino)-8-oxo-7,8-dihydropyrido[3,4-d]pyrimidin)-6-yl)phenyl)-3-(trifluoromethyl)benzamide;
(Compound No.5): N-(4-methyl-3-(7-methyl-2-((oxetan-2-ylmethyl)amino)-8-oxo-7,8-dihydropyrido[3,4-d]pyrimidin-6-yl)phenyl)-3-(trifluoromethyl)benzamide;
(Compound No. 6): N-(4-methyl-3-(7-methyl-2-((2-morpholinoethyl)amino)-8-oxo-7,8-dihydropyrido[3,4-d]pyrimidin-6-yl)phenyl)-3-(trifluoromethyl)benzamide;
(Compound No. 7): N-(4-methyl-3-(7-methyl-8-oxo-2-((2-(thiazol-2-yl)ethyl)amino)-7,8-dihydropyrido[3,4-d]pyrimidin-6-yl)phenyl)-3-(trifluoromethyl)benzamide;
(Compound No. 8): N-(4-methyl-3-(7-methyl-8-oxo-2-((2-(thiophen-2-yl)ethyl)amino)-7,8-dihydropyrido[3,4-d]pyrimidin-6-yl)phenyl)-3-(trifluoromethyl)benzamide;
(Compound No. 9): N-(4-methyl-3-(7-methyl-2-((2-(4-nitrophenoxy)ethyl)amino)-8-oxo-7,8-dihydropyrido[3,4-d]pyrimidin-6-yl)phenyl)-3-(trifluoromethyl)benzamide;
(Compound No. 10): N-(3-(2-((4-methoxybenzyl)amino)-7-methyl-8-oxo-7,8-dihydropyrido[3,4-d]pyrimidin-6-yl)-4-methylphenyl)-3-(trifluoromethyl)benzamide;
(Compound No. 11): N-(3-(2-((2-((furan-2-ylmethyl)thio)ethyl)amino)-7-methyl-8-oxo-7,8-dihydiropyrido[3,4-d]pyrimidin-6-yl)-4-methylphenyl)-3-(trifluoromethyl)benzamide;
(Compound No. 12): N-(4-methyl-3-(7-methyl-2-morpholino-8-oxo-7,8-dihydropyrido[3,4-d]pyrimidin-6-yl)phenyl)-3-(trifluoromethyl)benzamide;
(Compound No. 13): N-(4-methyl-3-(7-methyl-2-((6-methylpyridin-3-yl)amino)-8-oxo-7,8-dihydropyrido[3,4-d]pyrimidin-6-yl)phenyl)-3-(trifluoromethyl)benzamide;
(Compound No. 14): N-(4-methyl-3-(7-methyl-8-oxo-2-(phenylamino)-7,8-dihydropyrido[3,4-d]pyrimidin-6-yl)phenyl)-3-(trifluoromethyl)benzamide;
(Compound No. 15): N,N-dimethyl-4-((7-methyl-6-(2-methyl-5-(3-(trifluoromethyl)benzamido)phenyl)-8-oxo-7,8-dihydropyrimido[3,4-d]pyrimidin-2-yl)amino)benzamide;
(Compound No. 16): N-(4-methyl-3-(7-methyl-2-((1-methyl-1H-pyrazol-4-yl)amino)-8-oxo-7,8-dihydropyrido[3,4-d]pyrimidin-6-yl)phenyl)-3-(trifluoromethyl)benzamide;
(Compound No. 17): N-(3-(2-((4-(4-ethylpiperazin-1-yl)phenyl)amino)-7-methyl-8-oxo-7,8-dihydropyrido[3,4-d]pyrimidin-6-yl)-4-methylphenyl)-3-(trifluoromethyl)benzamide;
(Compound No. 18): N-(3-(2-((3-(4-ethylpiperazin-1-yl)phenyl)amino)-7-methyl-8-oxo-7,8-dihydropyrido[3,4-d]pyrimidin-6-yl)-4-methylphenyl)-3-(trifluoromethyl)benzamide;
(Compound No. 19): N-(3-(2-((4-(4-hydroxypiperidin-1-yl)phenyl)amino)-7-methyl-8-oxo-7,8-dihydropyrido[3,4-d]pyrimidin-6-yl)-4-methylphenyl)-3-(trifluoromethyl)benzamide;
(Compound No. 20): N-(4-methyl-3-(7-methyl-2-((6-morpholinopyridin-3-yl)amino)-8-oxo-7,8-dihydropyrido[3,4-d]pyrimidin-6-yl)phenyl)-3-(trifluoromethyl)benzamide;
(Compound No. 21): N-(4-methyl-3-(7-methyl-2-((6-(4-(4-methylpiperazin-1-yl)piperidin-1-yl)pyridin3-yl)amino)-8-oxo-7,8-dihydropyrido[3,4-d]pyrimidin-6-yl)phenyl)-3-(trifluoromethyl)benzamide;
(Compound No. 22): N-(3-(2-((2-methoxy-4-morpholinophenyl)amino)-7-methyl-8-oxo-7,8-dihydropyrido[3,4-d]pyrimidin-6-yl)-4-methylphenyl)-3-(trifluoromethyl)benzamide;
(Compound No. 23): N-(4-methyl-3-(7-methyl-2-((4-((4-methylpiperazin-1-yl)methyl)-3-(trifluoromethyl)phenyl)amino)-8-oxo-7,8-dihydropyrido[3,4-d]pyrimidin-6-yl)phenyl)-3-(trifluoromethyl)benzamide;
(Compound No. 24): N-(4-methyl-3-(7-methyl-2-((6-methylpyridin-3-yl)amino)-8-oxo-7,8-dihydropyrido[3,4-d]pyrimidin-6-yl)phenyl)benzamide;
(Compound No. 25): 5-methyl-N-(4-methyl-3-(7-methyl-2-((6-methylpyridin-3-yl)amino)-8-oxo-7,8-dihydropyrido[3,4-d]pyrimidin-6-yl)phenyl)nicotinamide;
(Compound No. 26): N-(4-methyl-3-(7-methyl-2-((6-methylpyridin-3-yl)amino)-8-oxo-7,8-dihydiropyrido[3,4-d]pyrimidin-6-yl)phenyl)thiophene-3-carboxamide;
(Compound No. 27): N-(4-methyl-3-(7-methyl-2-((6-methylpyridin-3-yl)amino)-8-oxo-7,8-dihydropyrido[3,4-d]pyrimidin-6-yl)phenyl)benzofuran-2-carboxamide;
(Compound No. 28): N-(4-methyl-3-(7-methyl-2-((6-methylpyridin-3-yl)amino)-8-oxo-7,8-dihydropyrido[3,4-d]pyrimidin-6-yl)phenyl)-2-naphthamide;
(Compound No. 29): N-(4-methyl-3-(7-methyl-2-((6-methylpyridin-3-yl)amino)-8-oxo-7,8-dihydropyrido[3,4-d]pyrimidin-6-yl)phenyl)-2,3-dihydrobenzo[b][1,4]dioxy-6-carboxamide;
(Compound No. 30): N-(4-methyl-3-(7-methyl-2-((6-methylpyridin-3-yl)amino)-8-oxo-7,8-dihydropyrido[3,4-d]pyrimidin-6-yl)phenyl)-2-(3-(trifluoromethyl)phenyl)acetamide;
(Compound No. 31): N-(4-methyl-3-(7-methyl-2-((6-methylpyridin-3-yl)amino)-8-oxo-7,8-dihydropyrido[3,4-d]pyrimidin-6-yl)phenyl) acetamide;
(Compound No. 32): N-(4-methyl-3-(7-methyl-2-((6-methylpyridin-3-yl)amino)-8-oxo-7,8-dihydropyrido[3,4-d]pyrimidin-6-yl)phenyl)-4-(4-methylpyperazin-1-yl)benzamide;
(Compound No. 33): N-(4-methyl-3-(7-methyl-2-((6-methylpyridin-3-yl)amino)-8-oxo-7,8-dihydropyrido[3,4-d]pyrimidin-6-yl)phenyl)propionamide;
(Compound No. 34): N-(4-methyl-3-(7-methyl-2-((6-methylpyridin-3-yl)amino)-8-oxo-7,8-dihydropyrido[3,4-d]pyrimidin-6-yl)phenyl)-4-morpholinobenzamide;
(Compound No. 35): N-(4-methyl-3-(7-methyl-2-((6-methylpyridin-3-yl)amino)-8-oxo-7,8-dihydropyrido[3,4-d]pyrimidin-6-yl)phenyl)-4-((4-methylpiperazin-1-yl)methyl)benzamide;
(Compound No. 36): N-(4-methyl-3-(7-methyl-2-((6-methylpyridin-3-yl)amino)-8-oxo-7,8-dihydropyrido[3,4-d]pyrimidin-6-yl)phenyl)-3-(4-methylpiperazin-1-yl)-5-(trifluoromethyl)benzamide;
(Compound No. 37): 3-(2,4-dimethyl-1H-imidazol-1-yl)-N-(4-methyl-3-(7-methyl-2-((6-methylpyridin-3-yl))amino)-8-oxo-7,8-dihydropyrido[3,4-d]pyrimidin-6-yl)phenyl)-5-(trifluoromethyl)benzamide;
(Compound No. 38): 3-(4-hydroxypiperidin-1-yl)-N-(4-methyl-3-(7-methyl-2-((6-methylpiperidin-3-yl)amino)-8-oxo-7,8-dihydropyrido[3,4-d]pyrimidin-6-yl)phenyl)-5-(trifluoromethyl)benzamide;
(Compound No. 39): 4-(4-methyl-1H-imidazol-1-yl)-N-(4-methyl-3-(7-methyl-2-((6-methylpyridin-3-yl)amino-8)-oxo-7,8-dihydropyrido[3,4-d]pyrimidin-6-yl)phenyl)-3-(trifluoromethyl)benzamide;
(Compound No. 40): N-(4-methyl-3-(7-methyl-2-((6-methylpyridin-3-yl)amino)-8-oxo-7,8-dihydropyrido[3,4-d]pyrimidin-6-yl)phenyl)-4-(4-methylpiperazin-1-yl)-3-(trifluoromethyl)benzamide;
(Compound No. 41): N-(4-methyl-3-(7-methyl-2-((6-methylpyridin-3-yl)amino)-8-oxo-7,8-dihydropyrido[3,4-d]pyrimidin-6-yl)phenyl)-4-(morpholinomethyl)-3-(trifluoromethyl)benzamide;
(Compound No. 42): 4-((3-(dimethylamino)pinolidin-1-yl)methyl)-N-(4-methyl-3-(7-methyl-2-((6-methylpyridine-3-yl)amino)-8-oxo-7,8-dihydropyrido[3,4-d]pyrimidin-6-yl)phenyl)-3-(trifluoromethyl)benzamide;
(Compound No. 43): N-(4-methyl-3-(7-methyl-2-((6-methylpyridin-3-yl)amino)-8-oxo-7,8-dihydropyrido[3,4-d]pyrimidin-6-yl)phenyl)methanesulfonamide;
(Compound No. 44): N-(4-methyl-3-(7-methyl-2-((6-methylpyridin-3-yl)amino)-8-oxo-7,8-dihydropyrido[3,4-d]pyrimidin-6-yl)phenyl)-2-nitrobenzsulfonamide;
(Compound No. 45): 3-bromo-N-(4-methyl-3-(7-methyl-2-((6-methylpyrimidin-3-yl)amino)-8-oxo-7,8-dihydropyrido[3,4-d]pyrimidin-6-yl)phenyl)benzenesulfonamide;
(Compound No. 46): 4-fluoro-N-(4-methyl-3-(7-methyl-2-((6-methylpyridin-3-yl)amino)-8-oxo-7,8-dihydropyrido[3,4-d]pyrimidin-6-yl)phenyl)benzenesulfonamide;
(Compound No. 47): 1-cyclohexyl-3-(4-methyl-3-(7-methyl-2-((6-methylpyridin-3-yl)amino)-8-oxo-7,8-dihydropyrido[3,4-d]pyrimidin-6-yl)phenyl)urea;
(Compound No. 48): 1-(2,3-dichlorophenyl)-3-(4-methyl-3-(7-methyl-2-((6-methylpyridin-3-yl)amino)-8-oxo-7,8-dihydropyrido[3,4-d]pyrimidin-6-yl)phenyl)urea;
(Compound No. 49): 1-(2-methoxyphenyl)-3-(4-methyl-3-(7-methyl-2-((6-methylpyridin-3-yl)amino)-8-oxo-7,8-dihydropyrido[3,4-d]pyrimidin-6-yl)phenyl)urea;
(Compound No. 50): 6-(5-(ethylamino)-2-methylphenyl)-7-methyl-2-((6-methylpyridin-3-yl)amino)pyrido[3,4-d]pyridin-8(7H)-one;
(Compound No. 51): 6-(5-((4-fluorobenzyl)amino)-2-methylphenyl)-7-methyl-2-((6-methylpyridin-3-yl)amino)pyrido[3,4-d]pyrimidin-8(7H)-one;
(Compound No. 52): 6-(5-(((5-bromofuran-2-yl)methyl)amino)-2-methylphenyl)-7-methyl-2-((6-methylpyridin-3-yl)amino)pyrido[3,4-d]pyrimidin-8(7H)-one;
(Compound No. 53): N- (3- (2- (cyclopropylamino) -8-oxo-7,8-dihydropyrido[3,4-d]pyrimidin-6-yl)-4-methylphenyl)-3-(trifluoromethyl)benzamide;
(Compound No. 54): N-(4-methyl-3-(2-((6-methylpyridin-3-yl)amino)-8-oxo-7,8-dihydropyrido[3,4-d]pyrimidin-6-yl)phenyl)-3-(trifluoromethyl)benzamide; and
(Compound No. 55): N-(3-(2-amino-8-oxo-7,8-dihydropyrido[3,4-d]pyrimidin-6-yl)-4-methylphenyl)-3-(trifluoromethyl)benzamide.

In the definition of substituents in the present disclosure, the term "'alkyl" refers to an aliphatic hydrocarbon radical. The alkyl may be saturated alkyl that does not contain an alkenyl or alkynyl moiety, or unsaturated alkyl that contains at least one alkenyl or alkynyl moiety. The term "alkenyl" refers to a group containing at least one carbon-carbon double bond, and the term "alkynyl" refers to a group containing at least one carbon-carbon triple bond. The alkyl may be cyclic, branched or straight-chain when used alone or in combination.

The term "aryl" as used herein, either alone or in combination with another radical, refers to a carbocyclic aromatic monocyclic group containing 6 carbon atoms, which may be further fused to a second 5- or 6-membered carbocyclic group which may be aromatic, saturated or unsaturated. Examples of aryl include, but are not limited to, phenyl, indanyl, 1-naphthyl, 2-naphthyl, and tetrahydronaphthyl. Aryl may be connected to another group at a suitable position on the aromatic ring.

The term "alkoxy" refers to an alkyl group attached via an oxygen atom to another group (i.e., -O-alkyl). The alkoxy group may be unsubstituted or substituted with one or more suitable substituents. Examples of the alkoxy group include, but are not limited to, (C₁-C₆) alkoxy groups, for example, -O-methyl, -O-ethyl, -O-propyl, -O-isopropyl, -O-2-methyl-1-propyl, -O-2-methyl-2-propyl, -O-2-methyl-1-butyl, -O-3-methyl-1-butyl, -O-2-methyl-3-butyl, -O-2,2-dimethyl-1-propyl, -O-2-methyl-1-pentyl, 3-O-methyl-1-pentyl, -O-4-methyl-1-pentyl, -O-2-methyl-2-pentyl, -O-3-methyl-2-pentyl, -O-4-methyl-2-pentyl, -O-2,2-dimethyl-1-butyl, -O-3,3-dimethyl-butyl, -O-2-ethyl-1-butyl, -O-butyl, -O-isobutyl, -O-t-butyl, -O-pentyl, -O-isopentyl, -O-neopentyl, and -O-hexyl.

The term "phenoxy" refers to a phenyl group attached via an oxygen atom to another group (i.e., -O-aryl). The phenoxy group may be unsubstituted or substituted with one or more halogens, alkyl groups, aryl groups or heteroaryl groups, but is not limited thereto.

The term "amino group" refers to an alkyl group attached via a nitrogen atom to another group (i.e., -NH- or -N-alkyl). The amino group may be unsubstituted or substituted with one or more suitable substituents. Examples of the amine group include, but are not limited to, (C₁-C₆) amino groups, for example, -NH-methyl, -NH-ethyl, - NH-propyl, -NH-isopropyl, -NH-2-methyl-1-propyl, -NH-2-methyl-2-propyl, -NH-2-methyl-1-butyl, -NH-3-methyl-1-butyl, -NH-2-methyl-3-butyl, -NH-2,2-dimethyl-1-propyl, -NH-2-methyl-1-pentyl, 3-NH-methyl-1-pentyl, -NH-4-methyl-1-pentyl, -NH-2-methyl-2-pentyl, -NH-3-methyl-2-pentyl, -NH-4-methyl-2-pentyl, -NH-2,2-dimethyl-1-butyl, -NH-3,3-dimethyl-butyl, -NH-2-ethyl-1-butyl, -NH-butyl, -NH-isobutyl, -NH-t-butyl, -NH-pentyl, -NH-isopentyl, -NH-neopentyl, -NH-hexyl, -N,N-dimethyl, -N-methyl-N-ethyl, -N-methyl-N-propyl, -N-methyl-isopropyl, -N-methyl-N-butyl, - N-methyl-N-isobutyl, -N-methyl-N-pentyl, -N-methyl-N-isopentyl, N-methyl-N-hexyl, N-methyl-N-isohexyl, -N,N-diethyl, -N-ethyl-N-propyl, -N-ethyl-N-isopropyl, -N-ethyl-N-butyl, -N-ethyl-N-isobutyl, -N-ethyl-N-pentyl, -N-ethyl-N-isopentyl, -N-ethyl-N-hexyl, -N-ethyl-N-isohexyl, -N,N-dipropyl, -N-propyl-N-isopropyl, -N-propyl-N-butyl, -N-propyl-N-isobutyl, -N-propyl-N-pentyl, -N-propyl-N-isopentyl, -N-propyl-N-hexyl, -N-propyl-N-isohexyl, -N,N-dibutyl, -N-butyl-N-isobutyl, -N-butyl-N-pentyl, - N-butyl-N-isopentyl, -N-butyl-N-hexyl, -N-butyl-N-isohexyl, -N,N-dipentyl, -N-pentyl-N-hexyl, -N-pentyl- N-isohexyl, and - N,N-dihexyl.

The term "halogen group" refers to fluorine, chlorine, bromine or iodine.

The term "heterocyclyl group" refers to a heteroaromatic compound containing at least one heteroatom selected from the group consisting of N, O and S, unless otherwise stated. Preferably, the heterocyclyl group may include, but is not limited to, a pyrrolidine group, a furan group, a morpholine group, a piperazine group and a piperidine group, more preferably a pyrrolidine group, a piperidine group, a piperazine group, and a morpholine group.

The term "heteroaryl group" refers to a heteroaromatic compound containing at least one heteroatom selected from the group consisting of N, O and S, unless otherwise stated. Preferably, the heteroaryl group may include, but is not limited to, a pyridine group, a pyrazine group, a pyrimidine group, a pyridazine group, a pyrazole group, an imidazole group, a triazole group, an indole group, an oxadiazole group, a thiadiazole group, a quinolone group, an isoquinoline group, an isoxazole group, an oxazole group, a thiazolyl group, and a pyrrole group.

Specific examples of preferred compounds as the compounds according to the present disclosure are as follows:
(Compound No. 1): N-(3-(2-(cyclopropylamino)-7-methyl-8-oxo-7,8-dihydropyrido[3,4-d]pyrimidin-6-yl)-4-methylphenyl)-3-(trifluoromethyl)benzamide 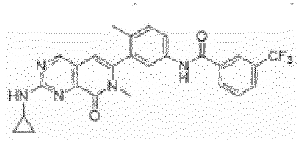
(Compound No. 2): N-(3-(2-((2-hydroxyethyl)amino)-7-methyl-8-oxo-7,8-dihydropyrido[3,4-d]pyrimidin-6-yl)-4-methylphenyl)-3-(trifluoromethyl)benzamide 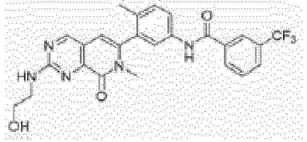
(Compound No. 3): N-(4-methyl-3-(7-methyl-2-(methylamino)-8-oxo-7,8-dihydropyrido[3,4-d]pyrimidin-6-yl)phenyl)-3-(trifluoromethyl)benzamide 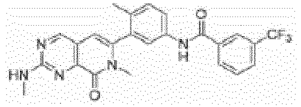
(Compound No. 4): N-(4-methyl-3-(7-methyl-2-(oxetan-3-ylamino)-8-oxo-7,8-dihydropyrido[3,4-d]pyrimidin)-6-yl)phenyl)-3-(trifluoromethyl)benzamide; 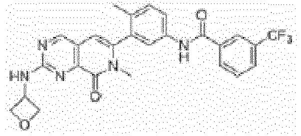
(Compound No.5): N-(4-methyl-3-(7-methyl-2-((oxetan-2-ylmethyl)amino)-8-oxo-7,8-dihydropyrido[3,4-d]pyrimidin-6-yl)phenyl)-3-(trifluoromethyl)benzamide 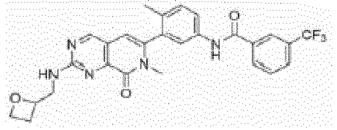
(Compound No. 6): N-(4-methyl-3-(7-methyl-2-((2-morpholinoethyl)amino)-8-oxo-7,8-dihydropyrido[3,4-d]pyrimidin-6-yl)phenyl)-3-(trifluoromethyl)benzamide 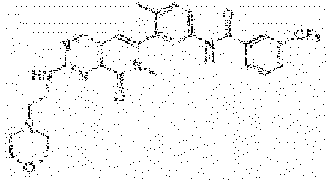
(Compound No. 7): N-(4-methyl-3-(7-methyl-8-oxo-2-((2-(thiazol-2-yl)ethyl)amino)-7,8-dihydropyrido[3,4-d]pyrimidin-6-yl)phenyl)-3-(trifluoromethyl)benzamide 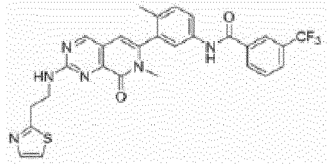
(Compound No. 8): N-(4-methyl-3-(7-methyl-8-oxo-2-((2-(thiophen-2-yl)ethyl)amino)-7,8-dihydropyrido[3,4-d]pyrimidin-6-yl)phenyl)-3-(trifluoromethyl)benzamide 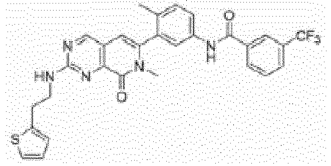
(Compound No. 9): N-(4-methyl-3-(7-methyl-2-((2-(4-nitrophenoxy)ethyl)amino)-8-oxo-7,8-dihydropyrido[3,4-d]pyrimidin-6-yl)phenyl)-3-(trifluoromethyl)benzamide 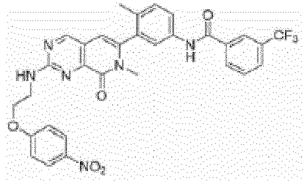
(Compound No. 10): N-(3-(2-((4-methoxybenzyl)amino)-7-methyl-8-oxo-7,8-dihydropyrido[3,4-d]pyrimidin-6-yl)-4-methylphenyl)-3-(trifluoromethyl)benzamide 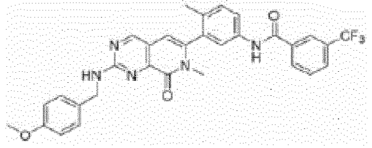
(Compound No. 11): N-(3-(2-((2-((furan-2-ylmethyl)thio)ethyl)amino)-7-methyl-8-oxo-7,8-dihydiropyrido[3,4-d]pyrimidin-6-yl)-4-methylphenyl)-3-(trifluoromethyl)benzamide 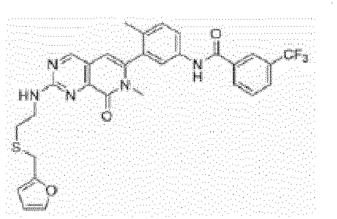
(Compound No. 12): N-(4-methyl-3-(7-methyl-2-morpholino-8-oxo-7,8-dihydropyrido[3,4-d]pyrimidin-6-yl)phenyl)-3-(trifluoromethyl)benzamide 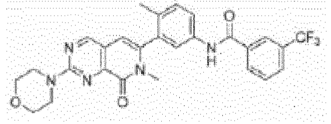
(Compound No. 13): N-(4-methyl-3-(7-methyl-2-((6-methylpyridin-3-yl)amino)-8-oxo-7,8-dihydropyrido[3,4-d]pyrimidin-6-yl)phenyl)-3-(trifluoromethyl)benzamide 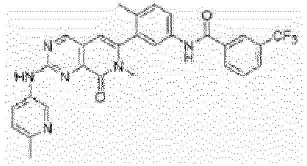
(Compound No. 14): N-(4-methyl-3-(7-methyl-8-oxo-2-(phenylamino)-7,8-dihydropyrido[3,4-d]pyrimidin-6-yl)phenyl)-3-(trifluoromethyl)benzamide 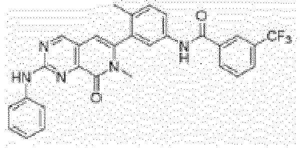
(Compound No. 15): N,N-dimethyl-4-((7-methyl-6-(2-methyl-5-(3-(trifluoromethyl)benzamido)phenyl)-8-oxo-7,8-dihydropyrimido[3,4-d]pyrimidin-2-yl)amino)benzamide 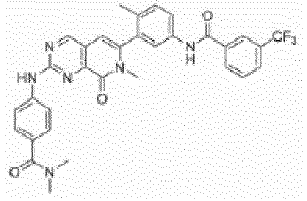
(Compound No. 16): N-(4-methyl-3-(7-methyl-2-((1-methyl-1H-pyrazol-4-yl)amino)-8-oxo-7,8-dihydropyrido[3,4-d]pyrimidin-6-yl)phenyl)-3-(trifluoromethyl)benzamide 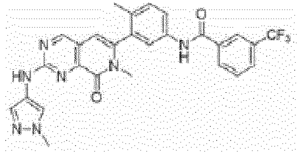
(Compound No. 17): N-(3-(2-((4-(4-ethylpiperazin-1-yl)phenyl)amino)-7-methyl-8-oxo-7,8-dihydropyrido[3,4-d]pyrimidin-6-yl)-4-methylphenyl)-3-(trifluoromethyl)benzamide 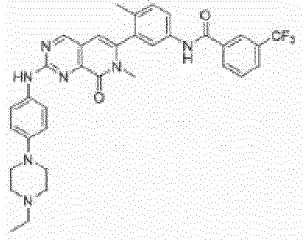
(Compound No. 18): N-(3-(2-((3-(4-ethylpiperazin-1-yl)phenyl)amino)-7-methyl-8-oxo-7,8-dihydropyrido[3,4-d]pyrimidin-6-yl)-4-methylphenyl)-3-(trifluoromethyl)benzamide 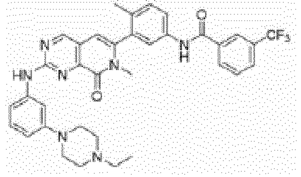
(Compound No. 19): N-(3-(2-((4-(4-hydroxypiperidin-1-yl)phenyl)amino)-7-methyl-8-oxo-7,8-dihydropyrido[3,4-d]pyrimidin-6-yl)-4-methylphenyl)-3-(trifluoromethyl)benzamide 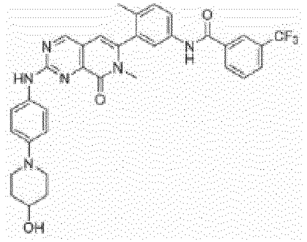
(Compound No. 20): N-(4-methyl-3-(7-methyl-2-((6-morpholinopyridin-3-yl)amino)-8-oxo-7,8-dihydropyrido[3,4-d]pyrimidin-6-yl)phenyl)-3-(trifluoromethyl)benzamide 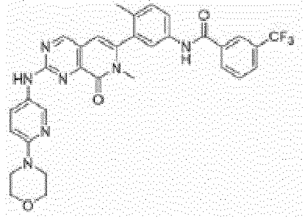
(Compound No. 21): N-(4-methyl-3-(7-methyl-2-((6-(4-(4-methylpiperazin-1-yl)piperidin-1-yl)pyridin3-yl)amino)-8-oxo-7,8-dihydropyrido[3,4-d]pyrimidin-6-yl)phenyl)-3-(trifluoromethyl)benzamide 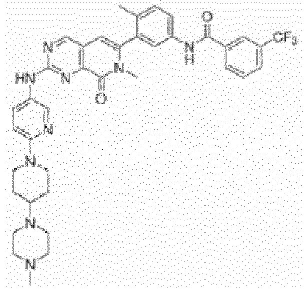
(Compound No. 22): N-(3-(2-((2-methoxy-4-morpholinophenyl)amino)-7-methyl-8-oxo-7,8-dihydropyrido[3,4-d]pyrimidin-6-yl)-4-methylphenyl)-3-(trifluoromethyl)benzamide 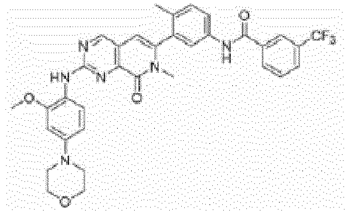
(Compound No. 23): N-(4-methyl-3-(7-methyl-2-((4-((4-methylpiperazin-1-yl)methyl)-3-(trifluoromethyl)phenyl)amino)-8-oxo-7,8-dihydropyrido[3,4-d]pyrimidin-6-yl)phenyl)-3-(trifluoromethyl)benzamide 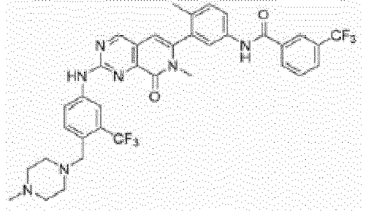
(Compound No. 24): N-(4-methyl-3-(7-methyl-2-((6-methylpyridin-3-yl)amino)-8-oxo-7,8-dihydropyrido[3,4-d]pyrimidin-6-yl)phenyl)benzamide 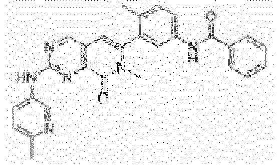
(Compound No. 25): 5-methyl-N-(4-methyl-3-(7-methyl-2-((6-methylpyridin-3-yl)amino)-8-oxo-7,8-dihydropyrido[3,4-d]pyrimidin-6-yl)phenyl)nicotinamide 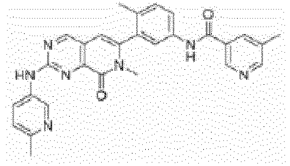
(Compound No. 26): N-(4-methyl-3-(7-methyl-2-((6-methylpyridin-3-yl)amino)-8-oxo-7,8-dihydiropyrido[3,4-d]pyrimidin-6-yl)phenyl)thiophene-3-carboxamide 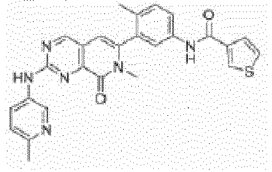
(Compound No. 27): N-(4-methyl-3-(7-methyl-2-((6-methylpyridin-3-yl)amino)-8-oxo-7,8-dihydropyrido[3,4-d]pyrimidin-6-yl)phenyl)benzofuran-2-carboxamide 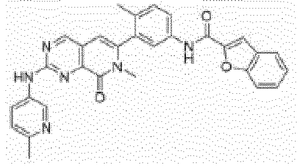
(Compound No. 28): N-(4-methyl-3-(7-methyl-2-((6-methylpyridin-3-yl)amino)-8-oxo-7,8-dihydropyrido[3,4-d]pyrimidin-6-yl)phenyl)-2-naphthamide 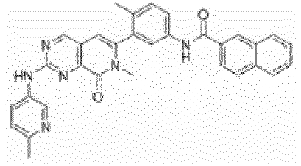
(Compound No. 29): N-(4-methyl-3-(7-methyl-2-((6-methylpyridin-3-yl)amino)-8-oxo-7,8-dihydropyrido[3,4-d]pyrimidin-6-yl)phenyl)-2,3-dihydrobenzo[b] [1,4]dioxy-6-carboxamide 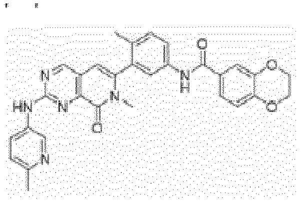
(Compound No. 30): N-(4-methyl-3-(7-methyl-2-((6-methylpyridin-3-yl)amino)-8-oxo-7,8-dihydropyrido[3,4-d]pyrimidin-6-yl)phenyl)-2-(3-(trifluoromethyl)phenyl)acetamide 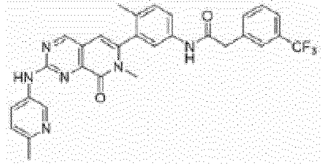
(Compound No. 31): N-(4-methyl-3-(7-methyl-2-((6-methylpyridin-3-yl)amino)-8-oxo-7,8-dihydropyrido[3,4-d]pyrimidin-6-yl)phenyl)acetamide 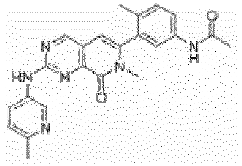
(Compound No. 32): N-(4-methyl-3-(7-methyl-2-((6-methylpyridin-3-yl)amino)-8-oxo-7,8-dihydropyrido[3,4-d]pyrimidin-6-yl)phenyl)-4-(4-methylpyperazin-1-yl)benzamide 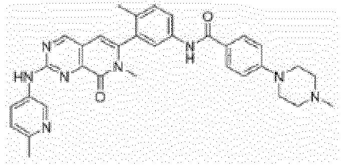
(Compound No. 33): N-(4-methyl-3-(7-methyl-2-((6-methylpyridin-3-yl)amino)-8-oxo-7,8-dihydropyrido[3,4-d]pyrimidin-6-yl)phenyl)propionamide 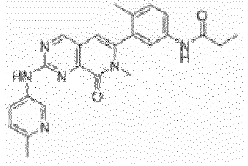
(Compound No. 34): N-(4-methyl-3-(7-methyl-2-((6-methylpyridin-3-yl)amino)-8-oxo-7,8-dihydropyrido[3,4-d]pyrimidin-6-yl)phenyl)-4-morpholinobenzamide 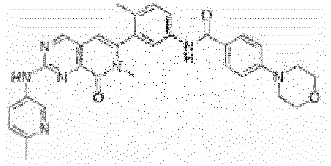
(Compound No. 35): N-(4-methyl-3-(7-methyl-2-((6-methylpyridin-3-yl)amino)-8-oxo-7,8-dihydropyrido[3,4-d]pyrimidin-6-yl)phenyl)-4-((4-methylpiperazin-1-yl)methyl)benzamide 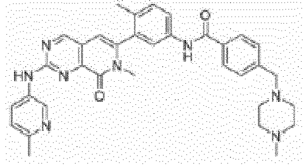
(Compound No. 36): N-(4-methyl-3-(7-methyl-2-((6-methylpyridin-3-yl)amino)-8-oxo-7,8-dihydropyrido[3,4-d]pyrimidin-6-yl)phenyl)-3-(4-methylpiperazin-1-yl)-5-(trifluoromethyl)benzamide 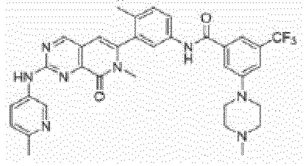
(Compound No. 37): 3-(2,4-dimethyl-1H-imidazol-1-yl)-N-(4-methyl-3-(7-methyl-2-((6-methylpyridin-3-yl))amino)-8-oxo-7,8-dihydropyrido[3,4-d]pyrimidin-6-yl)phenyl)-5-(trifluoromethyl)benzamide 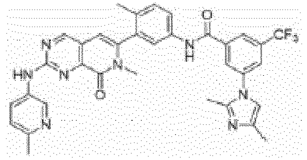
(Compound No. 38): 3-(4-hydroxypiperidin-1-yl)-N-(4-methyl-3-(7-methyl-2-((6-methylpiperidin-3-yl)amino)-8-oxo-7,8-dihydropyrido[3,4-d]pyrimidin-6-yl)phenyl)-5-(trifluoromethyl)benzamide 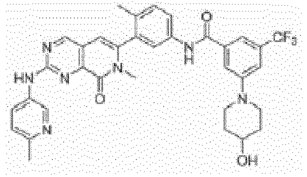
(Compound No. 39): 4-(4-methyl-1H-imidazol-1-yl)-N-(4-methyl-3-(7-methyl-2-((6-methylpyridin-3-yl)amino-8)-oxo-7,8-dihydropyrido[3,4-d]pyrimidin-6-yl)phenyl)-3-(trifluoromethyl)benzamide 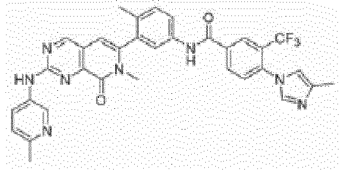
(Compound No. 40): N-(4-methyl-3-(7-methyl-2-((6-methylpyridin-3-yl)amino)-8-oxo-7,8-dihydropyrido[3,4-d]pyrimidin-6-yl)phenyl)-4-(4-methylpiperazin-1-yl)-3-(trifluoromethyl)benzamide 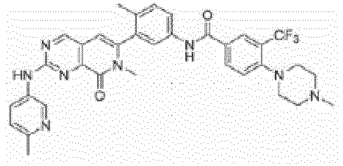
(Compound No. 41): N-(4-methyl-3-(7-methyl-2-((6-methylpyridin-3-yl)amino)-8-oxo-7,8-dihydropyrido[3,4-d]pyrimidin-6-yl)phenyl)-4-(morpholinomethyl)-3-(trifluoromethyl)benzamide 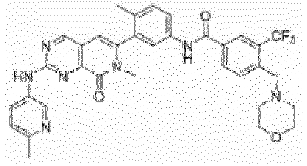
(Compound No. 42): 4-((3-(dimethylamino)pinolidin-1-yl)methyl)-N-(4-methyl-3-(7-methyl-2-((6-methylpyridine-3-yl)amino)-8-oxo-7,8-dihydropyrido[3,4-d]pyrimidin-6-yl)phenyl)-3-(trifluoromethyl)benzamide 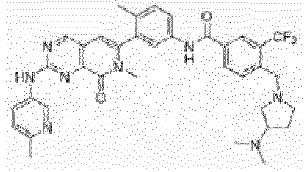
(Compound No. 43): N-(4-methyl-3-(7-methyl-2-((6-methylpyridin-3-yl)amino)-8-oxo-7,8-dihydropyrido[3,4-d]pyrimidin-6-yl)phenyl)methanesulfonamide 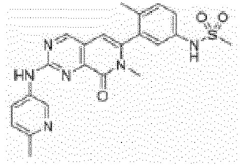
(Compound No. 44): N-(4-methyl-3-(7-methyl-2-((6-methylpyridin-3-yl)amino)-8-oxo-7,8-dihydropyrido[3,4-d]pyrimidin-6-yl)phenyl)-2-nitrobenzsulfonamide 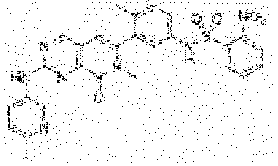
(Compound No. 45): 3-bromo-N-(4-methyl-3-(7-methyl-2-((6-methylpyrimidin-3-yl)amino)-8-oxo-7,8-dihydropyrido[3,4-d]pyrimidin-6-yl)phenyl)benzenesulfonamide 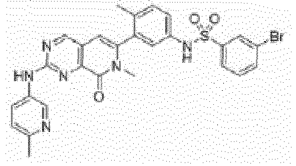
(Compound No. 46): 4-fluoro-N-(4-methyl-3-(7-methyl-2-((6-methylpyridin-3-yl)amino)-8-oxo-7,8-dihydropyrido[3,4-d]pyrimidin-6-yl)phenyl)benzenesulfonamide 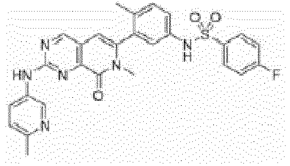
(Compound No. 47): 1-cyclohexyl-3-(4-methyl-3-(7-methyl-2-((6-methylpyridin-3-yl)amino)-8-oxo-7,8-dihydropyrido[3,4-d]pyrimidin-6-yl)phenyl)urea 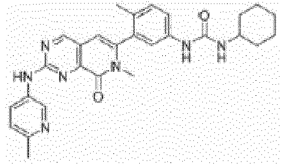
(Compound No. 48): 1-(2,3-dichlorophenyl)-3-(4-methyl-3-(7-methyl-2-((6-methylpyridin-3-yl)amino)-8-oxo-7,8-dihydropyrido[3,4-d]pyrimidin-6-yl)phenyl)urea 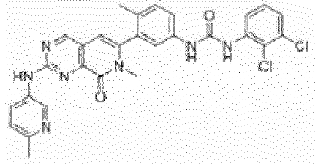
(Compound No. 49): 1-(2-methoxyphenyl)-3-(4-methyl-3-(7-methyl-2-((6-methylpyridin-3-yl)amino)-8-oxo-7,8-dihydropyrido[3,4-d]pyrimidin-6-yl)phenyl)urea 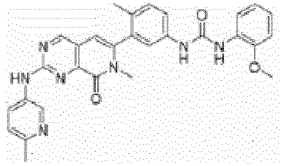
(Compound No. 50): 6-(5-(ethylamino)-2-methylphenyl)-7-methyl-2-((6-methylpyridin-3-yl)amino)pyrido[3,4-d]pyridin-8(7H)-one 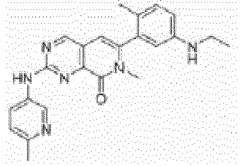
(Compound No. 51): 6-(5-((4-fluorobenzyl)amino)-2-methylphenyl)-7-methyl-2-((6-methylpyridin-3-yl)amino)pyrido[3,4-d]pyrimidin-8(7H)-one 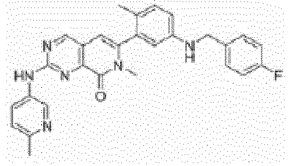
(Compound No. 52): 6-(5-(((5-bromofuran-2-yl)methyl)amino)-2-methylphenyl)-7-methyl-2-((6-methylpyridin-3-yl)amino)pyrido[3,4-d]pyrimidin-8(7H)-one 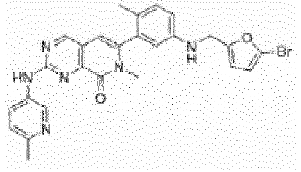
(Compound No. 53): N-(3-(2-(cyclopropylamino)-8-oxo-7,8-dihydropyrido[3,4-d]pyrimidin-6-yl)-4-methylphenyl)-3-(trifluoromethyl)benzamide 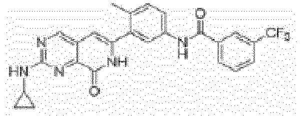
(Compound No. 54): N-(4-methyl-3-(2-((6-methylpyridin-3-yl)amino)-8-oxo-7,8-dihydropyrido[3,4-d]pyrimidin-6-yl)phenyl)-3-(trifluoromethyl)benzamide 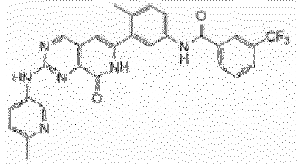
(Compound No. 55): N-(3-(2-amino-8-oxo-7,8-dihydropyrido[3,4-d]pyrimidin-6-yl)-4-methylphenyl)-3-(trifluoromethyl)benzamide

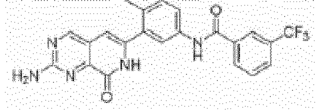

The compound of Formula 1 according to the present disclosure may be used in the form of a pharmaceutically acceptable salt derived from an inorganic acid or an organic acid. A preferred salt may be formed with one or more selected from the group consisting of hydrochloric acid, hydrobromic acid, sulfuric acid, phosphoric acid, nitric acid, acetic acid, glycolic acid, lactic acid, pyruvic acid, malonic acid, succinic acid, glutaric acid, fumaric acid, malic acid, mandelic acid, tartaric acid, citric acid, ascorbic acid, palmitic acid, maleic acid, hydroxymaleic acid, benzoic acid, hydroxybenzoic acid, phenylacetic acid, cinnamic acid, salicylic acid, methanesulfonic acid, benzenesulfonic acid, and toluenesulfonic acid.

The compound of Formula 1 according to the present disclosure or a pharmaceutically acceptable salt thereof may include a hydrate and a solvate. The hydrate may refer to one formed by the combination of the compound of Formula 1 with a water molecule.

Another aspect of the present disclosure provides a pharmaceutical composition for preventing, alleviating or treating cancer containing, as an active ingredient, a compound selected from among the compound of Formula 1 according to the present disclosure, a pharmaceutically acceptable salt thereof, a hydrate thereof, and a stereoisomer thereof.

The pharmaceutical composition according to the present disclosure has excellent ability to inhibit the activity of protein kinase. Specific examples of the protein kinase include ABL1, FGFR2, TAOK2/TA01, EPHA5, EPHB2, EPHB3, RET, LYN B, EPHA2, FRK/PTK5, EPHA8, LCK, EPHB4, FYN, KHS/MAP4K5, DDR1, EPHA3, P38a/MAPK14, EPHA4, FMS, EPHB1, HCK, FGFR1, ABL2/ARG, EPHA6, c-Src, ACK1, FLT4/VEGFR3, ERBB4/HER4, DDR2, KDR/VEGFR2, LYN, ZAK/MLTK, YES/YES1, BLK, FGR, MLCK2/MYLK2, TAOK1, BMX/ETK, BTK, EPHA1, JAK1, P38b/MAPK11, TIE2/TEK, FLT1/VEGFR1, TXK, SRMS, RAF1, SIK1, MLK3/MAP3K11, PEAK1, TRKA, EPHA7, GLK/MAP4K3, MLK2/MAP3K10, TEC, CSK, TRKC, FES/FPS, SIK2, FGFR3, BRK, YSK4/MAP3K19, ARAF, PDGFRb, TNK1, GCK/MAP4K2, PDGFRa, TNIK, TAK1, ERBB2/HER2, LIMK1, HIPK4, FER, EGFR, JAK2, HPK1/MAP4K1, TRKB, RIPK3, LOK/STK10, LIMK2, MLK1/MAP3K9, BRAF, MEKK3, MEK5, STK32B/YANK2, FGFR4, MEKK2, SLK/STK2, FLT3, PKAcg, TAOK3/JIK, TYRO3/SKY, SIK3, IR, LRRK2, PYK2, NEK11, p70S6K/RPS6KB1, and LATS2.

Thus, the pharmaceutical composition of the present disclosure may be used for the purpose of treating, preventing and alleviating cancer disease caused by abnormal cell growth. Cancer diseases that may be treated, prevented and alleviated by treatment with the pharmaceutical composition according to the present disclosure include stomach cancer, lung cancer, liver cancer, colorectal cancer, small intestine cancer, pancreatic cancer, brain cancer, bone cancer, melanoma, breast cancer, sclerosing adenosis, uterine cancer, cervical cancer, head and neck cancer, esophageal cancer, thyroid cancer, parathyroid cancer, kidney cancer, sarcoma, prostate cancer, urethral cancer, bladder cancer, blood cancer (including leukemia, multiple myeloma, and myelodysplastic syndrome), lymphoma (Hodgkin's disease, and non-Hodgkin's lymphoma), psoriasis, or fibroadenoma.

In particular, the pharmaceutical composition of the present disclosure has inhibitory activity against two kinases, GCK and ACK1, and thus is effective for the treatment of cancer diseases caused by NRAS mutation, for example, melanoma, colorectal cancer, thyroid cancer, or various blood cancers. In addition, the compound represented by Formula 1 exhibits inhibitory activity against the proliferation of the NRAS mutant cell line (OCI-AML3) without showing inhibitory activity against the Ba/F3 (parental) cell line, and thus is particularly effective as a therapeutic agent for treating acute myeloid leukemia (AML).

] Preferably, the cancer may be cancer caused by a protein kinase. More preferably, the protein kinase may be at least one selected from among GCK and ACK1.

Another aspect of the present disclosure provides a pharmaceutical composition for preventing, alleviating or treating cancer, the pharmaceutical composition containing, as an active ingredient, any one compound selected from among the above-described compounds.

In another aspect of the present disclosure, there is provided a pharmaceutical composition for preventing, alleviating or treating cancer, wherein the cancer is caused by NRAS mutation.

In another aspect of the present disclosure, there is provided a pharmaceutical composition for preventing, alleviating or treating cancer, wherein the pharmaceutical composition is applied to patients with NRAS mutation.

In another aspect of the present disclosure, there is provided a pharmaceutical composition for preventing, alleviating or treating cancer, wherein the cancer is cancer is at least one selected from the group consisting of melanoma, colorectal cancer, thyroid cancer, and blood cancer.

In another aspect of the present disclosure, there is provided a pharmaceutical composition for preventing, alleviating or treating cancer, wherein the cancer is acute myeloid leukemia (AML).

In another aspect of the present disclosure, there is provided a pharmaceutical composition for preventing, alleviating or treating cancer, wherein the pharmaceutical composition is administered to a patient with NRAS G12D.

The pharmaceutical composition may be applied to experimental animals such as mice, rabbits, rats, guinea pigs, or hamsters, or primates including humans, but is not limited thereto. Preferably, the pharmaceutical composition may be applied to primates including humans, more preferably humans.

In the present disclosure, the term "treating" or "treatment" may be used in a sense including alleviation or amelioration of symptoms, reduction of the range of disease, delay or alleviation of disease progression, amelioration, alleviation or stabilization of disease conditions, partial or complete recovery, prolonging of survival, and other beneficial therapeutic outcomes.

In addition, the treatment of cancer as used in the present specification refers to treatment of all cancer cell types, and the term "cancer" also includes angiogenesis of endothelial cells and mitosis thereof (solid tumors, tumor metastases and benign tumors). Examples of the cancer include, but are not limited to, breast cancer, ovarian cancer, cervical cancer, prostate cancer, testicular cancer, genitourinary tract cancer, esophageal cancer, laryngeal cancer, glioblastoma, stomach cancer, skin cancer, keratoacanthoma, lung cancer, squamous cell carcinoma, large cell carcinoma, small cell carcinoma, lung adenocarcinoma, bone cancer, colon cancer, adenoma, pancreatic cancer, adenocarcinoma, thyroid cancer, follicular adenocarcinoma, undifferentiated cancer, papillary cancer, seminoma, melanoma, sarcoma, bladder cancer, liver and bile duct cancer, kidney cancer, myeloid disease, lymphoid disease, Hodgkin's disease, hair cell cancer, oral cancer, pharyngeal (laryngeal) cancer, lip cancer, tongue cancer, small intestine cancer, colorectal cancer, large intestine cancer, rectal cancer, brain cancer, central nervous system cancer, leukemia, angioma, trachoma, or pyogenic granuloma.

Depending on the aspect and method of use of the pharmaceutical composition of the present disclosure, the content of the compound represented by Formula 1, a pharmaceutically acceptable salt thereof, or a hydrate thereof, which is an active ingredient, may be appropriately selected and adjusted by those skilled in the art.

For example, the pharmaceutical composition may contain the compound represented by Formula 1, a pharmaceutically acceptable salt thereof, or a hydrate thereof in an amount of 0.1 to 10 wt%, more preferably 0.5 to 5 wr% by weight, based on the total weight of the composition.

The compound represented by Formula 1, a pharmaceutically acceptable salt thereof, or a hydrate thereof may be contained alone in the pharmaceutical composition or may also be contained together with a pharmaceutically acceptable carrier, excipient, diluent or accessory ingredient.

Examples of the pharmaceutically acceptable carrier, excipient or diluent include, but are not limited to, at least one selected from the group consisting of lactose, dextrose, sucrose, sorbitol, mannitol, xylitol, erythritol, maltitol, starch, gum acacia, alginate, gelatin, calcium phosphate, calcium silicate, cellulose, methyl cellulose, microcrystalline cellulose, polyvinyl pyrrolidone, water, methylhydroxybenzoate, propylhydroxybenzoate, talc, magnesium stearate, mineral oil, dextrin, calcium carbonate, propylene glycol, liquid paraffin, and physiological saline. In addition, any conventional carrier, excipient or diluent may also be used. In addition, the pharmaceutical composition may further contain a conventional filler, extender, binder, disintegrant, anti-aggregating agent, lubricant, wetting agent, pH-adjusting agent, nutrient, vitamin, electrolyte, alginic acid and its salt, pectic acid and its salt, protective colloid, glycerin, fragrance, emulsifier or preservative.

The compound of Formula 1 according to the present disclosure or a pharmaceutically acceptable salt thereof may be co-administered with other anticancer drugs for treating cancer or tumors, thus enhancing the therapeutic effects of the anticancer drugs.

Specifically, the pharmaceutical composition may further contain one or more other anticancer drugs or other therapeutic agents known to be effective for the treatment or prevention of cancer, in addition to the active ingredient, and may be used in combination therapy in which they are applied simultaneously or at different times. For example, other anticancer drugs or other therapeutic agents that may be applied to the combination therapy may include, but are not limited to, one or more compounds selected from the group consisting of Gleevec^{®} (imatinib), Sutent^{®} (sunitinib), Herceptin^{®} (Trastuzumab), Velcade^{®} (Bortezomib), dexamethasone, Nexavar^{®} (Sorafenib), aromatase inhibitors, and kinase inhibitors.

The mode of administration of the pharmaceutical composition may be oral or parenteral. For example, the pharmaceutical composition may be administered through various routes, including oral, transdermal, subcutaneous, intravenous or intramuscular routes. In addition, the formulation of the composition may vary depending on the method of use thereof, and the composition may be formulated using a method well known in the art to which the present disclosure pertains, so as to provide rapid, sustained or delayed release of the active ingredient after administration to a mammal. In general, solid preparations for oral administration include tablets, troches, soft or hard capsules, pills, powders, and granules, and these formulations may be prepared by mixing one or more excipients, for example, starch, calcium carbonate, sucrose, lactose, or gelatin. In addition to simple excipients, lubricants such as magnesium stearate and talc may also be used. Liquid formulations for oral administration include suspensions, liquids for internal use, emulsions, and syrups, which may contain various excipients, for example, wetting agents, sweetening agents, fragrances, preservatives, and the like, in addition to water and liquid paraffin, which are commonly used simple diluents. Dosage forms for parenteral administration include cream, lotions, ointments, plasters, liquids, solutions, aerosols, fluid extracts, elixirs, infusions, sachets, patches, or injections. When the dosage form for parenteral administration is a dosage form for injection, it may preferably be in the form of an isotonic aqueous solution or suspension.

The pharmaceutical composition may further contain a sterilizing agent, a preservative, a stabilizer, a wetting agent or an emulsifying agent, adjuvants such as a salt and/or buffer for regulating osmotic pressure, and other therapeutically useful substances, and may be formulated according to a conventional mixing, granulation or coating method. In addition, the pharmaceutical composition may be formulated using an appropriate method known in the art.

In addition, the dosage of the pharmaceutical composition may be determined in consideration of the mode of administration, the patient's age and sex, the patient's disease severity and condition, the *in vivo* absorption rate of the active ingredient, the rate of inactivation, and drugs to be used in combination, and may be administered once or several times. The active ingredient of the pharmaceutical composition may preferably administered to mammals including humans once or several times a day by an oral or parenteral route at a dose of 0.001 to 100 mg/kg body weight/day, preferably 0.01 to 35 mg/kg body weight/day.

Another embodiment of the present disclosure provides a method for treating cancer, the method comprising a step of administering a therapeutically effective amount of the compound represented by Formula 1, a pharmaceutically acceptable salt thereof, or a hydrate thereof.

Preferably, the treatment method may further comprise a step of identifying a patient in need of the prevention or treatment of the cancer, before the administering step.

In the present disclosure, the term "therapeutically effective amount" refers to an amount of the active ingredient for a mammal, which is effective for the prevention or treatment of cancer. The therapeutically effective amount may be adjusted depending on various factors, including the kind of disease, the severity of the disease, the kinds and contents of the active ingredient and other ingredients contained in the composition, the type of formulation, the patient's age, weight, general health status, sex and diet, the time of administration, the route of administration, the blood clearance of the composition, the duration of treatment, and drugs that are used concurrently. Preferably, as described above, the active ingredient may be administered once or several times a day by an oral or parenteral route at a dose of 0.001 to 100 mg/kg body weight/day, preferably 0.01 to 35 mg/kg body weight/day.

In addition, the present disclosure is directed to a method for producing the pyrido[3,4-d]pyrimidin-8-one derivative compound represented by Formula 1, a pharmaceutically acceptable salt thereof, or a hydrate thereof.

### Mode for Invention

Hereinafter, the present disclosure will be described in detail with reference to examples and experimental examples. However, the following examples and experimental examples serve to merely illustrate the present disclosure, and the scope of the present disclosure is not limited by the following examples.

### [Examples] Methods for producing pyrido[3,4-d]pyrimidin-8-one derivative compounds of Formula 1

### Example 1: N-(3-(2-(cyclopropylamino)-7-methyl-8-oxo-7,8-dihydropyrido[3,4-d]pyrimidin-6-yl)-4-methylphenyl)-3-(trifluoromethyl)benzamide

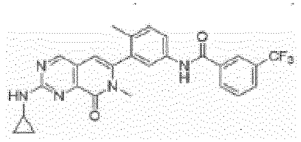

### Step 1: Methyl 5-bromo-2-(methylthio)pyrimidine-4-carboxylate

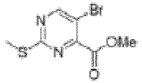

Acetyl chloride (1.1 eq., 10.5 mL) was dissolved in methanol (200 ml) at 0°C. After stirring at 0°C for 10 minutes, 5-bromo-2-(methylthio)pyrimidine-4-carboxylic acid (25 g, 1 eq.) was added slowly over 15 minutes. The reaction solution was refluxed for 4 hours and cooled. After completion of the reaction, the reaction solution was concentrated under reduced pressure. The concentrate was diluted with dichloromethane, and then washed with a saturated aqueous solution of sodium bicarbonate and then with brine. The organic layer was dried with magnesium sulfate and concentrated. The concentrate was recrystallized from ethyl acetate and hexane and then filtered to obtain the title compound (20 g, 76% yield) which was yellow in color. ¹H-NMR: (400 MHz, CDCl₃): δ (ppm) : 8.68 (1H, s), 3.98 (3H, s), 2.54 (3H, s). LCMS (ESI): 263 (M + H)⁺.

### Step 2: Trimethyl((2-methyl-5-nitrophenyl)ethynyl)silane

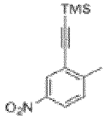

In a round bottom flask, 2-bromo-1-methyl-4-nitrobenzene (15 g, 1 eq.), diisopropylethylamine (24 mL, 2 eq.), Pd(PPh₃)₄ (4 g, 0.05 eq)., cuprous iodide (1.3 g, 0.1 eq.) and trimethylsilylacetylene (12 mL, 1.2 eq.) were dissolved in dimethylformamide (50 mL). After stirring at 80°C for 4 hours, the reaction solution was filtered through celite. The filtrate was diluted with ethyl acetate and then washed with brine. The organic layer was dried with magnesium sulfate and concentrated. The concentrate was purified by chromatography (2% ethyl acetate/hexane) to obtain the title compound (10.5 g, 65% yield) which was brown in color. ¹H-NMR: (400 MHz, CD6CO): δ (ppm): 8.18 (1H, s), 8.14 (1H, d, J = 8.5 Hz), 7.58 (1H, d, J = 8.5 Hz), 2.54 (3H, s), 0.28 (9H, s).

### Step 3: 2-ethynyl-1-methyl-4-nitrobenzene

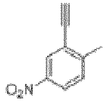

Calcium carbonate (15 g, 2.5 eq.) and methanol (100 mL) were placed in a round bottom flask, and trimethyl((2-methyl-5-nitrophenyl)ethynyl)silane (10.0 g, 1 eq.) was added slowly thereto dropwise. After completion of the reaction, the reaction solution was filtered through celite. The filtrate was diluted with ethyl acetate, and then washed with a saturated aqueous solution of ammonium chloride and then with brine. The organic layer was dried with magnesium sulfate and concentrated. The concentrate was purified by chromatography (2% ethyl acetate/hexane) to obtain the title compound (5.5 g, 80% yield) which was black in color. ¹H NMR (400 MHz, CD₆CO): δ (ppm): 8.23 (1H, s), 8.15 (1H, d, J = 8.5 Hz), 7.57 (1H, d, J = 8.5 Hz), 4.13 (1H, s), 2.55 (3H, s).

### Step 4: Methyl 5-((2-methyl-5-nitrophenyl)ethynyl)-2-(methylthio)pyrimidine-4-carboxylate

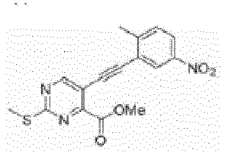

Methyl 5-bromo-2-(methylthio)pyrimididine-4-carboxylate (5 g, 1 eq.), 2-ethynyl-1-methyl-4-nitrobenzene (4.5 g, 1.2 eq.), PdCl₂(PPh₃)₂ (670 mg, 0.05 eq.) and cuprous iodide (362 mg, 0.1 eq.) were placed in a round bottom flask. Triethylamine as a solvent was added thereto, and then the reaction solution was stirred at 80°C for 25 hours. After completion of the reaction, the reaction solution was filtered through celite. The filtrate was diluted with dichloromethane and then washed with brine. The organic layer was dried with magnesium sulfate and concentrated. The concentrate was purified by chromatography (5% ethyl acetate/hexane) to obtain the title compound (5 g, 77% yield) which was white in color. ¹H-NMR: (400 MHz, CD₆CO): δ (ppm): 9.06 (1H, s), 8.35 (1H, s), 8.19 (1H, d, J = 8.5 Hz), 7.64 (1H, d, J = 8.5 Hz), 4.02 (3H, s), 2.69 (3H, s), 2.63 (3H, s). LCMS (ESI): 344 (M + H)⁺**.**

### Step 5: 5-((2-methyl-5-nitrophenyl)ethynyl)-2-(methylthio)pyrimidine-4-carboxylic acid

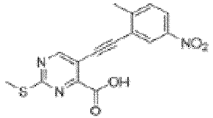

Methyl 5-((2-methyl-5-nitrophenyl)ethynyl)-2-(methylthio)pyrimidine-4-carboxylate (5 g, 1 eq.) was placed in a round bottom flask and dissolved slowly by the addition of tetrahydrofuran (50 mL). At 0°C, a saturated aqueous solution (40 mL) of 2 N sodium hydroxide was added slowly thereto dropwise. The reaction solution was stirred for 24 hours, and then tetrahydrofuran was removed under reduced pressure. The residue was adjusted to a pH of 3 to 4 using hydrogen chloride, and the formed solid was filtered and washed with water (2 x 50 mL). The washed solid was dried to obtain the title compound (3.4 g, 97% yield) which was yellow in color. ¹H-NMR: (400 MHz, DMSO-d6): δ (ppm): 9.08 (1H, s), 8.27 (1H, s), 8.17 (1H, d, J = 8.5 Hz), 7.61 (1H, d, J = 8.5 Hz), 3.50 (1H, brs), 2.59 (3H, s), 2.57 (3H, s). LCMS (ESI): 330 (M + H)⁺**.**

### Step 6: 6-(2-methyl-5-nitrophenyl)-2-(methylthio)-8H-pyrano[3,4-d]pyrimidin-8-one

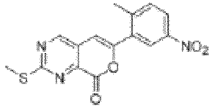

5-((2-methyl-5-nitrophenyl)ethynyl)-2-(methylthio)pyrimidine-4-carboxylic acid (3.4 g, 1 eq.) was placed in a round bottom flask and dissolved by the addition of toluene (25 mL). p-toluenesulfonic acid monohydrate (500 mg, 0.8 eq.) was added thereto, followed by stirring at 110°C for 7 hours. After completion of the reaction, the reaction solution was diluted with 10% methanol/dichloromethane, and then washed with a saturated aqueous solution of sodium bicarbonate and then with brine. The organic layer was dried with magnesium sulfate and concentrated. The concentrate was purified by chromatography (50% ethyl acetate/hexane) to obtain the title compound (2.4 g, 71% yield) which was yellow in color. ¹H-NMR: (400 MHz, CD₆CO): δ (ppm): 9.20 (1H, s), 8.45 (1H, s), 8.29 (1H, d, J = 8.5 Hz), 7.71 (1H, d, J = 8.5 Hz), 7.18 (1H, s), 2.70 (3H, s), 2.68 (3H, s). LCMS (ESI): 330 (M + H)⁺.

### Step 7: 6-(2-methyl-5-nitrophenyl)-2-(methylthio)pyrido[3,4-d]pyrimidin-8(7H)-one

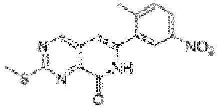

6-(2-methyl-5-nitrophenyl)-2-(methylthio)-8H-pyrano[3,4-d]pyrimidin-8-one (2.4 g, 1 eq.), acetic acid (25 mL) and ammonium acetate (5.6 g, 10 eq.) were placed in a round bottom flask and stirred at 90°C for 12 hours. After acetic acid was removed under reduced pressure, the residue was diluted with 20% isopropanol/chloroform. The dilution was washed with a saturated aqueous solution of sodium bicarbonate and then washed with brine. The organic layer was dried with magnesium sulfate and concentrated to obtain the title compound (2.2 g, 92% yield) which was brown in color. ¹H-NMR: (400 MHz, DMSO*d₆*): δ (ppm): 12.34 (1H, brs), 9.22 (1H, s), 8.72 (1H, s), 8.23 (1H, d, J = 8.5 Hz), 7.63 (1H, d, J = 8.5 Hz), 6.67 (1H, s), 2.63 (3H, s), 2.44 (3H, s). LCMS (ESI): 329 (M + H)⁺**.**

### Step 8: 7-methyl-6-(2-methyl-5-nitrophenyl)-2-(methylthiol)pyrido[3,4-d]pyrimidin-8(7H)-one

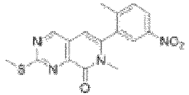

6-(2-methyl-5-nitrophenyl)-2-(methylthio)pyrido[3,4-d]pyrimidin-8(7H)-one (3.3 g, 1 eq.), methyl iodide (2 mL, 3 eq.), potassium carbonate (5.5 g, 4 eq.) and acetonitrile (50 mL) were placed in a round bottom flask and stirred at 80°C for 2 hours. After completion of the reaction, the reaction solution was filtered through celite and washed with 20% isopropanol/chloroform. The resulting material was concentrated under reduced pressure and purified by chromatography (2% methanol/dichloromethane) to obtain the title compound (2.7 g, 75% yield) which was yellow in color. LCMS (ESI): 343 (M + H)⁺**.**

### Step 9: 6-(5-amino-2-methylphenyl)-7-methyl-2-(methylthio)pyrido[3,4-d]pyrimidin-8(7H)-one

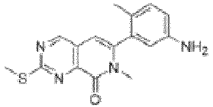

7-methyl-6-(2-methyl-5-nitrophenyl)-2-(methylthiol)pyrido[3,4-d]pyrimidin-8(7H)-one (300 mg, 1 eq.), iron (500 mg, 10 eq.), tetrahydrofuran:methanol (2:1, 15 mL) and an aqueous ammonium chloride solution (5 mL) were placed in in a round bottom flask and stirred at 80°C for 1 hour. After completion of the reaction, the reaction solution was filtered through celite and washed with 20% isopropanol/chloroform. The resulting material was washed with a saturated aqueous solution of sodium bicarbonate and then with brine. The organic layer was dried with magnesium sulfate and concentrated. The concentrate was recrystallized from ethyl ether, filtered, and dried to obtain the title compound (191 mg, 70% yield) which was yellow in color. LCMS (ESI): 313 (M + H)⁺**.**

### Step 10: N-(4-methyl-3-(7-methyl-2-(methylthio)-8-oxo-7,8-dihydropyrido[3,4-d]pyrimidin-6-yl)phenyl)-3-(trifluoromethyl)benzamide

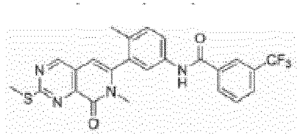

6-(5-amino-2-methylphenyl)-7-methyl-2-(methylthio)pyrido[3,4-d]pyrimidin-8(7H)-one (100 mg, 1 eq.), diisopropylethylamine (0.2 mL, 3 eq.) and tetrahydrofuran:dichloromethane (4:1, 15 mL) were placed in a round bottom flask. At 0°C, 3-trifluoromethyl benzoic chloride (0.06 ml, 1.5 eq.) was added thereto, flowed by stirring at room temperature for 2 hours. After completion of the reaction, the reaction solution was diluted with dichloromethane, and then washed with a saturated aqueous solution of sodium bicarbonate and then with brine. The organic layer was dried with magnesium sulfate and concentrated. The concentrate was recrystallized from ethyl ether, filtered, and dried to obtain the title compound (130 mg, 85% yield) which was yellow in color. LCMS (ESI): 485 (M + H)⁺**.**

### Step 11: N-(4-methyl-3-(7-methyl-2-(methylsulfonyl)-8-oxo-7,8-dihydropyrido[3,4-d]pyrimidin-6-yl)phenyl)-3-(trifluoromethyl)benzamide

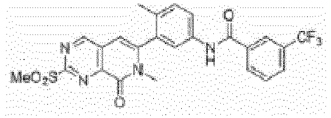

N-(4-methyl-3-(7-methyl-2-(methylthio)-8-oxo-7,8-dihydropyrido[3,4-d]pyrimidin-6-yl)phenyl)-3-(trifluoromethyl)benzamide (100 mg, 1 eq.) and dichloromethane (10 mL) were placed in a round bottom flask. At 0°C, 3-chloroperbenzoic acid (70 mg, 2.5 eq.) was added thereto, followed by stirring at room temperature for 1 hour. After completion of the reaction, the reaction solution was diluted with 20% isopropanol/chloroform, and then washed with a saturated aqueous solution of sodium bicarbonate and then with brine. The organic layer was dried with magnesium sulfate and concentrated. The concentrate was recrystallized from ethyl ether, filtered, and dried to obtain the title compound (80 mg, 75% yield) which was yellow in color. LCMS (ESI): 517 (M + H)⁺**.**

### Step 12: N-(3-(2-(cyclopropylamino)-7-methyl-8-oxo-7,8-dihydropyrido[3,4-d]pyrimidin-6-yl)-4-methylphenyl)-3-(trifluoromethyl)benzamide

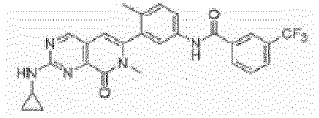

N-(4-methyl-3-(7-methyl-2-(methylsulfonyl)-8-oxo-7,8-dihydropyrido[3,4-d]pyrimidin-6-yl)phenyl)-3-(trifluoromethyl)benzamide (50 mg, 1 eq.), cyclopropylamine (2 eq.) and dimethylformamide (2 mL) were placed in a round bottom flask and stirred at 80°C for 2 hours. After completion of the reaction, the reaction solution was concentrated under reduced pressure and purified by chromatography to obtain the title compound which was yellow in color. LCMS (ESI): 494 (M + H)⁺**.**

### Example 2: N-(3-(2-((2-hydroxyethyl)amino)-7-methyl-8-oxo-7,8-dihydropyrido[3,4-d]pyrimidin-6-yl)-4-methylphenyl)-3-(trifluoromethyl)benzamide

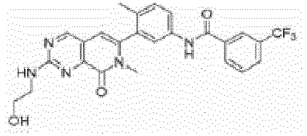

The experimental method was the same as that in Example 1, except that 2-aminoethanol was used instead of cyclopropylamine in step 12. LCMS (ESI): 498 (M + H)⁺**.**

### Example 3: N-(4-methyl-3-(7-methyl-2-(methylamino)-8-oxo-7,8-dihydropyrido[3,4-d]pyrimidin-6-yl)phenyl)-3-(trifluoromethyl)benzamide

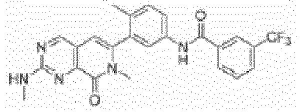

The experimental method was the same as that in Example 1, except that methylamine was used instead of cyclopropylamine in step 12. LCMS (ESI): 468 (M + H)⁺**.**

### Example 4: N-(4-methyl-3-(7-methyl-2-(oxetan-3-ylamino)-8-oxo-7,8-dihydropyrido[3,4-d]pyrimidin)-6-yl)phenyl)-3-(trifluoromethyl)benzamide

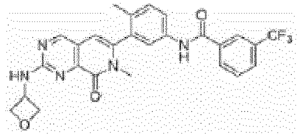

The experimental method was the same as that in Example 1, except that oxetan-3-amine was used instead of cyclopropylamine in step 12. LCMS (ESI): 510 (M + H)⁺**.**

### Example 5: N-(4-methyl-3-(7-methyl-2-((oxetan-2-ylmethyl)amino)-8-oxo-7,8-dihydropyrido[3,4-d]pyrimidin-6-yl)phenyl)-3-(trifluoromethyl)benzamide

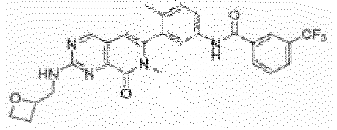

The experimental method was the same as that in Example 1, except that oxetan-3-ylmethanamine was used instead of cyclopropylamine in step 12. LCMS (ESI) : 524 (M + H)⁺**.**

### Example 6: N-(4-methyl-3-(7-methyl-2-((2-morpholinoethyl)amino)-8-oxo-7,8-dihydropyrido[3,4-d]pyrimidin-6-yl)phenyl)-3-(trifluoromethyl)benzamide

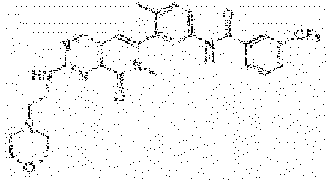

The experimental method was the same as that in Example 1, except that 2-morpholinoethan-1-amine was used instead of cyclopropylamine in step 12. LCMS (ESI): 567 (M + H)⁺.

### Example 7: N-(4-methyl-3-(7-methyl-8-oxo-2-((2-(thiazol-2-yl)ethyl)amino)-7,8-dihydropyrido[3,4-d]pyrimidin-6-yl)phenyl)-3-(trifluoromethyl)benzamide

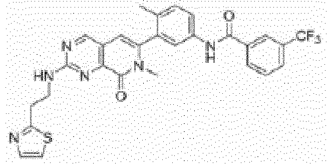

The experimental method was the same as that in Example 1, except that 2-(thiazol-2-yl)ethan-1-amine was used instead of cyclopropylamine in step 12. LCMS (ESI): 565 (M + H)⁺**.**

### Example 8: N-(4-methyl-3-(7-methyl-8-oxo-2-((2-(thiophen-2-yl)ethyl)amino)-7,8-dihydropyrido[3,4-d]pyrimidin-6-yl)phenyl)-3-(trifluoromethyl)benzamide

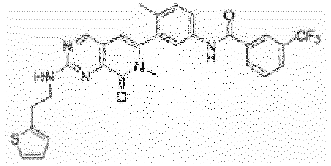

The experimental method was the same as that in Example 1, except that 2-(thiophen-2-yl)ethan-1-amine was used instead of cyclopropylamine in step 12. LCMS (ESI): 564 (M + H)⁺**.**

### Example 9: N-(4-methyl-3-(7-methyl-2-((2-(4-nitrophenoxy)ethyl)amino)-8-oxo-7,8-dihydropyrido[3,4-d]pyrimidin-6-yl)phenyl)-3-(trifluoromethyl)benzamide

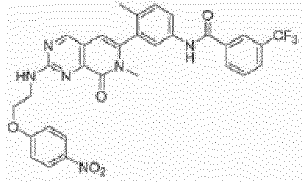

The experimental method was the same as that in Example 1, except that 2-(4-nitrophenoxy)ethan-1-amine was used instead of cyclopropylamine in step 12. LCMS (ESI): 619 (M + H)⁺.

### Example 10: N-(3-(2-((4-methoxybenzyl)amino)-7-methyl-8-oxo-7,8-dihydropyrido[3,4-d]pyrimidin-6-yl)-4-methylphenyl)-3-(trifluoromethyl)benzamide

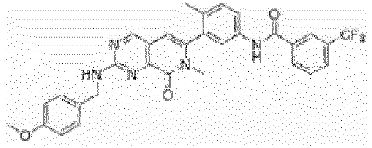

The experimental method was the same as that in Example 1, except that (4-methoxyphenyl)methanamine was used instead of cyclopropylamine in step 12. LCMS (ESI): 574 (M + H)⁺**.**

### Example 11: N-(3-(2-((2-((furan-2-ylmethyl)thio)ethyl)amino)-7-methyl-8-oxo-7,8-dihydiropyrido[3,4-d]pyrimidin-6-yl)-4-methylphenyl)-3-(trifluoromethyl)benzamide

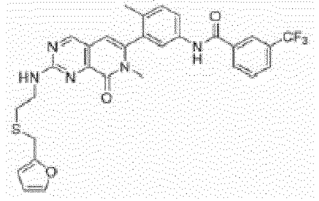

The experimental method was the same as that in Example 1, except that 2-((furan-2-ylmethyl)thio)ethan-1-amine was used instead of cyclopropylamine in step 12. LCMS (ESI): 594 (M +H)⁺**.**

### Example 12: N-(4-methyl-3-(7-methyl-2-morpholino-8-oxo-7,8-dihydropyrido[3,4-d]pyrimidin-6-yl)phenyl)-3-(trifluoromethyl)benzamide

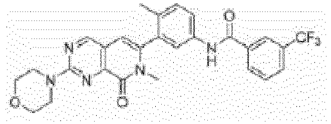

The experimental method was the same as that in Example 1, except that morpholine was used instead of cyclopropylamine in step 12. LCMS (ESI): 524 (M + H)⁺**.**

### Example 13: N-(4-methyl-3-(7-methyl-2-((6-methylpyridin-3-yl)amino)-8-oxo-7,8-dihydropyrido[3,4-d]pyrimidin-6-yl)phenyl)-3-(trifluoromethyl)benzamide

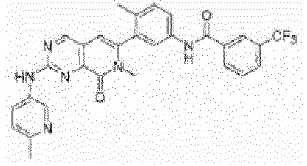

N-(4-methyl-3-(7-methyl-2-(methylsulfonyl)-8-oxo-7,8-dihydropyrido[3,4-d]pyrimidine-6-yl)phenyl)-3-(trifluoromethyl)benzamide (50 mg, 1 eq.) of step 11, N-(6-methylpyridin-3-yl)formamide (2 eq.), cesium carbonate (3 eq.) and dimethylformamide (2 mL) were placed in a round bottom flask and stirred at 80°C for 2 hours. After completion of the reaction, the reaction solution was concentrated under reduced pressure and purified by chromatography to obtain the title compound which was yellow in color. LCMS (ESI): 545 (M + H)⁺**.**

### Example 14: N-(4-methyl-3-(7-methyl-8-oxo-2-(phenylamino)-7,8-dihydropyrido[3,4-d]pyrimidin-6-yl)phenyl)-3-(trifluoromethyl)benzamide

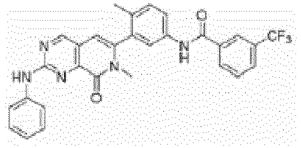

The experimental method was the same as that in Example 13, except that N-phenylformamide was used instead of N-(6-methylpyridin-3-yl) formamide. LCMS (ESI): 530 (M + H)⁺.

### Example 15: N,N-dimethyl-4-((7-methyl-6-(2-methyl-5-(3-(trifluoromethyl)benzamido)phenyl)-8-oxo-7,8-dihydropyrimido[3,4-d]pyrimidin-2-yl)amino)benzamide

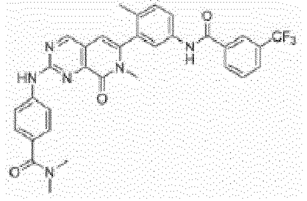

The experimental method was the same as that in Example 13, except that 4-formamido-N,N-dimethylbenzamide was used instead of N-(6-methylpyridin-3-yl)formamide. LCMS (ESI): 601 (M + H)⁺.

### Example 16: N-(4-methyl-3-(7-methyl-2-((1-methyl-1H-pyrazol-4-yl)amino)-8-oxo-7,8-dihydropyrido[3,4-d]pyrimidin-6-yl)phenyl)-3-(trifluoromethyl)benzamide

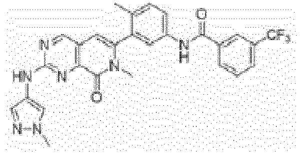

The experimental method was the same as that in Example 13, except that N-(1-methyl-1H-pyrazol-4-yl)formamide was used instead of N-(6-methylpyridin-3-yl)formamide.

### Example 17: N-(3-(2-((4-(4-ethylpiperazin-1-yl)phenyl)amino)-7-methyl-8-oxo-7,8-dihydropyrido[3,4-d]pyrimidin-6-yl)-4-methylphenyl)-3-(trifluoromethyl)benzamide

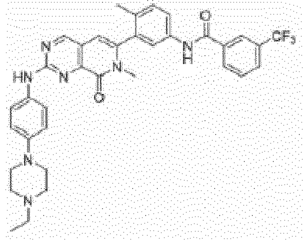

The experimental method was the same as that in Example 13, except that N-(4-(4-ethylpiperazin-1-yl)phenyl)formamide was used instead of N-(6-methylpyridin-3-yl)formamide. LCMS (ESI): 642 (M + H)⁺**.**

### Example 18: N-(3-(2-((3-(4-ethylpiperazin-1-yl)phenyl)amino)-7-methyl-8-oxo-7,8-dihydropyrido[3,4-d]pyrimidin-6-yl)-4-methylphenyl)-3-(trifluoromethyl)benzamide

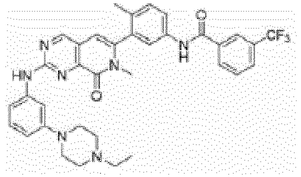

The experimental method was the same as that in Example 13, except that N-(3-(4-ethylpiperazin-1-yl)phenyl)formamide was used instead of N-(6-methylpyridin-3-yl)formamide. LCMS (ESI): 642 (M + H)⁺**.**

### Example 19: N-(3-(2-((4-(4-hydroxypiperidin-1-yl)phenyl)amino)-7-methyl-8-oxo-7,8-dihydropyrido[3,4-d]pyrimidin-6-yl)-4-methylphenyl)-3-(trifluoromethyl)benzamide

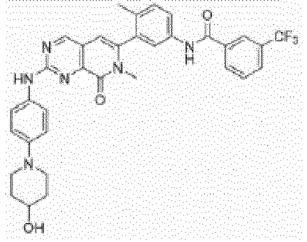

The experimental method was the same as that in Example 13, except that N-(4-(4-hydroxypiperidin-1-yl)phenyl)formamide was used instead of N-(6-methylpyridin-3-yl)formamide. LCMS (ESI): 630 (M + H)⁺**.**

### Example 20: N-(4-methyl-3-(7-methyl-2-((6-morpholinopyridin-3-yl)amino)-8-oxo-7,8-dihydropyrido[3,4-d]pyrimidin-6-yl)phenyl)-3-(trifluoromethyl)benzamide

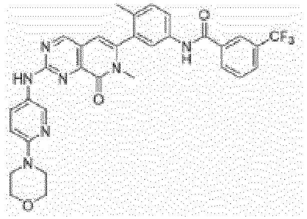

The experimental method was the same as that in Example 13, except that N-(6-morpholinopyridin-3-yl)formamide was used instead of N-(6-methylpyridin-3-yl)formamide. LCMS (ESI): 616 (M + H)⁺**.**

### Example 21: N-(4-methyl-3-(7-methyl-2-((6-(4-(4-methylpiperazin-1-yl)piperidin-1-yl)pyridin3-yl)amino)-8-oxo-7,8-dihydropyrido[3,4-d]pyrimidin-6-yl)phenyl)-3-(trifluoromethyl)benzamide

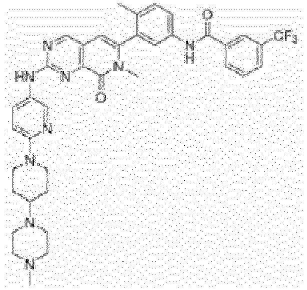

The experimental method was the same as that in Example 13, except that N-(6-(4-(4-methylpiperazin-1-yl)piperidin-1-yl)pyridin-3-yl)formamide was used instead of N-(6-methylpyridin-3-yl)formamide. LCMS (ESI): 712 (M + H)⁺.

### Example 22: N-(3-(2-((2-methoxy-4-morpholinophenyl)amino)-7-methyl-8-oxo-7,8-dihydropyrido[3,4-d]pyrimidin-6-yl)-4-methylphenyl)-3-(trifluoromethyl)benzamide

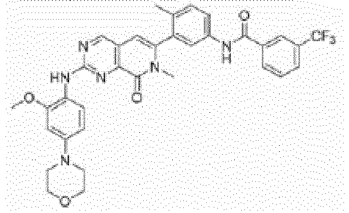

The experimental method was the same as that in Example 13, except that N-(2-methoxy-4-morpholinophenyl)formamide was used instead of N-(6-methylpyridin-3-yl)formamide. LCMS (ESI): 645 (M + H)⁺**.**

### Example 23: N-(4-methyl-3-(7-methyl-2-((4-((4-methylpiperazin-1-yl)methyl)-3-(trifluoromethyl)phenyl)amino)-8-oxo-7,8-dihydropyrido[3,4-d]pyrimidin-6-yl)phenyl)-3-(trifluoromethyl)benzamide

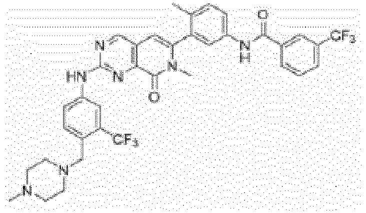

The experimental method was the same as that in Example 13, except that N-(4-((4-methylpiperazin-1-yl)methyl)-3-(trifluoromethyl)phenyl)formamide was used instead of N-(6-methylpyridin-3-yl)formamide. LCMS (ESI): 710 (M + H)⁺**.**

### Example 24: N-(4-methyl-3-(7-methyl-2-((6-methylpyridin-3-yl)amino)-8-oxo-7,8-dihydropyrido[3,4-d]pyrimidin-6-yl)phenyl)benzamide

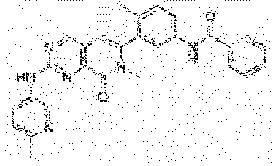

### Step 1: 7-methyl-6-(2-methyl-5-nitrophenyl)-2-(methylsulfonyl)pyrido[3,4-d]pyrimidin-8(7H)-one

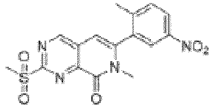

7-methyl-6-(2-methyl-5-nitrophenyl)-2-(methylthiol)pyrido[3,4-d]pyrimidin-8(7H)-one of step 8 (1.0 g, 1 eq.) was dissolved in dichloromethane (10 mL). At 0°C, 3-chloroperbenzoic acid (1.6 g, 3 eq.) was added thereto, followed by stirring at room temperature for 24 hours. After completion of the reaction, the reaction solution was diluted with 10% methanol/dichloromethane, and then washed with a saturated aqueous solution of sodium bicarbonate and then with brine. The organic layer was dried with magnesium sulfate and concentrated. The concentrate was purified by chromatography (0-10% methanol/dichloromethane) to obtain the title compound (550 mg, 50% yield) which was yellow in color. LCMS (ESI): 375 (M + H)⁺**.**

### Step 2: 7-methyl-6-(2-methyl-5-nitrophenyl)-2-((6-methylpyridin-3-yl)amino)pyrido[3,4-d]pyrimidin-8(7H)-one

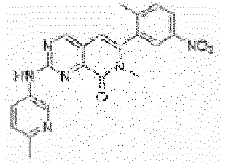

7-methyl-6-(2-methyl-5-nitrophenyl)-2-(methylsulfonyl)pyrido[3,4-d]pyrimidin-8(7H)-one (400 mg, 1 eq.), N-(6-methylpyrimidin-3-yl)formamide (225 mg, 1.5 eq.), cesium carbonate (1.81 g, 5 eq.) and dimethylsulfoxide (3 mL) were placed in a round bottom flask and stirred at 90°C for 2 hours. After completion of the reaction, the reaction solution was filtered through celite. The filtrate was diluted with ethyl acetate and then washed with brine. The organic layer was dried with magnesium sulfate and concentrated. The concentrate was purified by chromatography (0-10% methanol/dichloromethane) to obtain the title compound (120 mg, 28% yield) which was yellow in color. LCMS (ESI): 403 (M + H)⁺**.**

### Step 3: 6-(5-amino-2-methylphenyl)-7-methyl-2-((6-methylpyridin-3-yl)amino)pyrido[3,4-d]pyrimidin-8(7H)-one

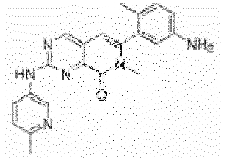

7-methyl-6-(2-methyl-5-nitrophenyl)-2-((6-methylpyridin-3-yl)amino)pyrido[3,4-d]pyrimidin-8(7H)-one (120 mg, 1 eq.), iron (168 mg, 10 eq.), ammonium chloride (318 mg, 10 eq.) and tetrahydrofuran:methanol:water (2:1:1 , 3 mL) were placed in a round bottom flask and stirred at 80°C for 1 hour. After completion of the reaction, the reaction solution was filtered through celite. The filtrate was washed with 20% methanol/dichloromethane, washed with a saturated aqueous solution of sodium bicarbonate, and then washed with brine. The organic layer was dried with magnesium sulfate and concentrated. The concentrate was purified by chromatography (0-10% methanol/dichloromethane) to obtain the title compound (95 mg, 85% yield) which was yellow in color. LCMS (ESI): 473 (M + H)⁺**.**

### Step 4: N-(4-methyl-3-(7-methyl-2-((6-methylpyridin-3-yl)amino)-8-oxo-7,8-dihydropyrido[3,4-d]pyrimidin-6-yl)phenyl)benzamide

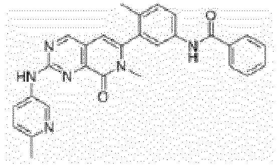

6-(5-amino-2-methylphenyl)-7-methyl-2-((6-methylpyridin-3-yl)amino)pyrido[3,4-d]pyrimidin-8(7H)-one (50 mg, 1 eq.), diisopropylethylamine (0.07 mL, 3 eq.) and tetrahydrofuran:dichloromethane (4:1, 3 mL) were placed in a round bottom flask. At 0°C, benzoic chloride (0.03 ml, 1.5 eq.) was added thereto, followed by stirring at room temperature for 2 hours. After completion of the reaction, the reaction solution was diluted with dichloromethane, and then washed with a saturated aqueous solution of sodium bicarbonate and then with brine. The organic layer was dried with magnesium sulfate and concentrated. The concentrate was purified by chromatography (0-10% methane/dichloromethane). LCMS (ESI): 477 (M + H)⁺**.**

### Example 25: 5-methyl-N-(4-methyl-3-(7-methyl-2-((6-methylpyridin-3-yl)amino)-8-oxo-7,8-dihydropyrido[3,4-d]pyrimidin-6-yl)phenyl)nicotinamide

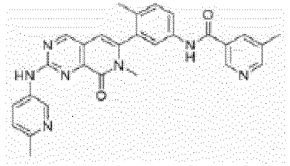

The experimental method was the same as that in Example 24, except that 5-methylnicotinoyl chloride was used instead of benzoic chloride in Step 4 of Example 24. LCMS (ESI): 492 (M + H)⁺**.**

### Example 26: N-(4-methyl-3-(7-methyl-2-((6-methylpyridin-3-yl)amino)-8-oxo-7,8-dihydiropyrido[3,4-d]pyrimidin-6-yl)phenyl)thiophene-3-carboxamide

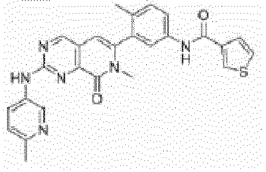

The experimental method was the same as that in Example 24, except that thiophene-3-carbonyl chloride was used instead of benzoic chloride in Step 4 of Example 24. LCMS (ESI): 483 (M + H)⁺**.**

### Example 27: N-(4-methyl-3-(7-methyl-2-((6-methylpyridin-3-yl)amino)-8-oxo-7,8-dihydropyrido[3,4-d]pyrimidin-6-yl)phenyl)benzofuran-2-carboxamide

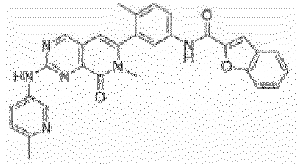

The experimental method was the same as that in Example 24, except that benzofuran-2-carbonyl chloride was used instead of benzoic chloride in Step 4 of Example 24. LCMS (ESI): 517 (M + H)⁺**.**

### Example 28: N-(4-methyl-3-(7-methyl-2-((6-methylpyridin-3-yl)amino)-8-oxo-7,8-dihydropyrido[3,4-d]pyrimidin-6-yl)phenyl)-2-naphthamide

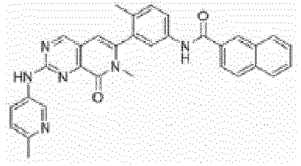

The experimental method was the same as that in Example 24, except that 2-naphthoyl chloride was used instead of benzoic chloride in Step 4 of Example 24. LCMS (ESI): 527 (M + H)⁺.

### Example 29: N-(4-methyl-3-(7-methyl-2-((6-methylpyridin-3-yl)amino)-8-oxo-7,8-dihydropyrido[3,4-d]pyrimidin-6-yl)phenyl)-2,3-dihydrobenzo[b] [1,4]dioxy-6-carboxamide

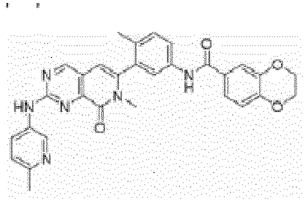

The experimental method was the same as that in Example 24, except that 2,3-dihydrobenzo[b][1,4]dioxy-6-carbonyl chloride was used instead of benzoic chloride in Step 4 of Example 24. LCMS (ESI): 535 (M + H)⁺**.**

### Example 30: N-(4-methyl-3-(7-methyl-2-((6-methylpyridin-3-yl)amino)-8-oxo-7,8-dihydropyrido[3,4-d]pyrimidin-6-yl)phenyl)-2-(3-(trifluoromethyl)phenyl)acetamide

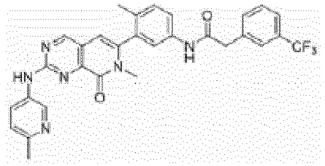

The experimental method was the same as that in Example 24, except that 2-(3-(trifluoromethyl)phenyl)acetyl chloride was used instead of benzoic chloride in Step 4 of Example 24. LCMS (ESI): 559 (M + H)⁺**.**

### Example 31: N-(4-methyl-3-(7-methyl-2-((6-methylpyridin-3-yl)amino)-8-oxo-7,8-dihydropyrido[3,4-d]pyrimidin-6-yl)phenyl)acetamide

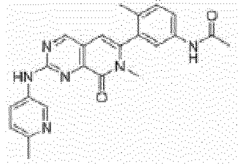

The experimental method was the same as that in Example 24, except that acetyl chloride was used instead of benzoic chloride in Step 4 of Example 24. LCMS (ESI): 415 (M + H)⁺.

### Example 32: N-(4-methyl-3-(7-methyl-2-((6-methylpyridin-3-yl)amino)-8-oxo-7,8-dihydropyrido[3,4-d]pyrimidin-6-yl)phenyl)-4-(4-methylpyperazin-1-yl)benzamide

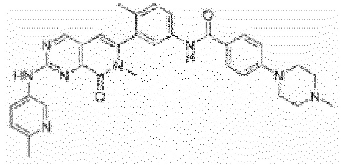

The experimental method was the same as that in Example 24, except that 4-(4-methylpyrezin-1-yl)benzoyl chloride was used instead of benzoic chloride in Step 4 of Example 24. LCMS (ESI): 575 (M + H)⁺**.**

### Example 33: N-(4-methyl-3-(7-methyl-2-((6-methylpyridin-3-yl)amino)-8-oxo-7,8-dihydropyrido[3,4-d]pyrimidin-6-yl)phenyl)propionamide

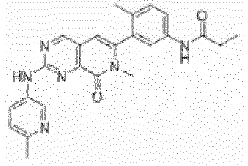

The experimental method was the same as that in Example 24, except that propionyl chloride was used instead of benzoic chloride in Step 4 of Example 24. LCMS (ESI): 429 (M + H)⁺.

**Example 34:** N-(4-methyl-3-(7-methyl-2-((6-methylpyridin-3-yl)amino)-8-oxo-7,8-dihydropyrido[3,4-d]pyrimidin-6-yl)phenyl)-4-morpholinobenzamide

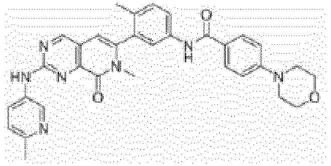

The experimental method was the same as that in Example 24, except that 4-morpholinobenzoyl chloride was used instead of benzoic chloride in Step 4 of Example 24. LCMS (ESI): 562 (M + H)⁺**.**

### Example 35: N-(4-methyl-3-(7-methyl-2-((6-methylpyridin-3-yl)amino)-8-oxo-7,8-dihydropyrido[3,4-d]pyrimidin-6-yl)phenyl)-4-((4-methylpiperazin-1-yl)methyl)benzamide

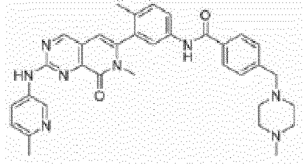

The experimental method was the same as that in Example 24, except that 4-((4-methylpiperazin-1-yl)methyl)benzoyl chloride was used instead of benzoic chloride in Step 4 of Example 24. LCMS (ESI): 589 (M + H)⁺**.**

### Example 36: N-(4-methyl-3-(7-methyl-2-((6-methylpyridin-3-yl)amino)-8-oxo-7,8-dihydropyrido[3,4-d]pyrimidin-6-yl)phenyl)-3-(4-methylpiperazin-1-yl)-5-(trifluoromethyl)benzamide

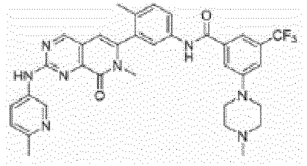

The experimental method was the same as that in Example 24, except that 3-(4-methylpiperazin-1-yl)-5-(trifluoromethyl)benzoyl chloride was used instead of benzoic chloride in Step 4 of Example 24. LCMS (ESI): 643 (M + H)⁺**.**

### Example 37: 3-(2,4-dimethyl-1H-imidazol-1-yl)-N-(4-methyl-3-(7-methyl-2-((6-methylpyridin-3-yl))amino)-8-oxo-7,8-dihydropyrido[3,4-d]pyrimidin-6-yl)phenyl)-5-(trifluoromethyl)benzamide

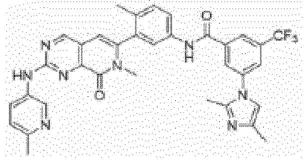

The experimental method was the same as that in Example 24, except that 3-(2,4-dimethyl-1H-imidazol-1-yl)-5-(trifluoromethyl)benzoyl chloride was used instead of benzoic chloride in Step 4 of Example 24. LCMS (ESI): 639 (M + H)⁺.

### Example 38: 3-(4-hydroxypiperidin-1-yl)-N-(4-methyl-3-(7-methyl-2-((6-methylpiperidin-3-yl)amino)-8-oxo-7,8-dihydropyrido[3,4-d]pyrimidin-6-yl)phenyl)-5-(trifluoromethyl)benzamide

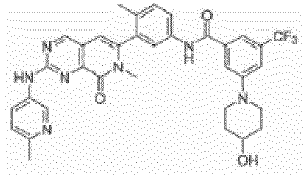

The experimental method was the same as that in Example 24, except that 3-(4-hydroxypiperidin-1-yl)-5-(trifluoromethyl)benzoyl chloride was used instead of benzoic chloride in Step 4 of Example 24. LCMS (ESI): 644 (M + H)⁺**.**

### Example 39: 4-(4-methyl-1H-imidazol-1-yl)-N-(4-methyl-3-(7-methyl-2-((6-methylpyridin-3-yl)amino-8)-oxo-7,8-dihydropyrido[3,4-d]pyrimidin-6-yl)phenyl)-3-(trifluoromethyl)benzamide

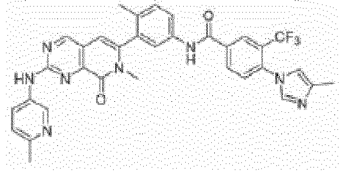

The experimental method was the same as that in Example 24, except that 4-(4-methyl-1H-imidazol-1-yl)-3-(trifluoromethyl)benzoyl chloride was used instead of benzoic chloride in Step 4 of Example 24. LCMS (ESI): 625 (M + H)⁺**.**

### Example 40: N-(4-methyl-3-(7-methyl-2-((6-methylpyridin-3-yl)amino)-8-oxo-7,8-dihydropyrido[3,4-d]pyrimidin-6-yl)phenyl)-4-(4-methylpiperazin-1-yl)-3-(trifluoromethyl)benzamide

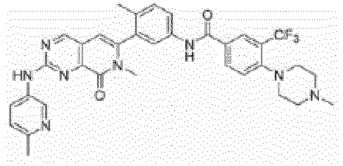

The experimental method was the same as that in Example 24, except that 4-(4-methylpiperazin-1-yl)-3-(trifluoromethyl)benzoyl chloride was used instead of benzoic chloride in Step 4 of Example 24. LCMS (ESI): 643 (M + H)⁺**.**

### Example 41: N-(4-methyl-3-(7-methyl-2-((6-methylpyridin-3-yl)amino)-8-oxo-7,8-dihydropyrido[3,4-d]pyrimidin-6-yl)phenyl)-4-(morpholinomethyl)-3-(trifluoromethyl)benzamide

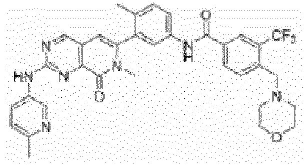

The experimental method was the same as that in Example 24, except that 4-(morpholinomethyl)-3-(trifluoromethyl)benzoyl chloride was used instead of benzoic chloride in Step 4 of Example 24. LCMS (ESI): 644 (M + H)⁺**.**

### Example 42: 4-((3-(dimethylamino)pinolidin-1-yl)methyl)-N-(4-methyl-3-(7-methyl-2-((6-methylpyridine-3-yl)amino)-8-oxo-7,8-dihydropyrido[3,4-d]pyrimidin-6-yl)phenyl)-3-(trifluoromethyl)benzamide

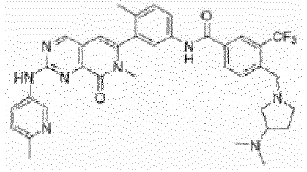

The experimental method was the same as that in Example 24, except that 4-((3-(dimethylamino)pyrrolidin-1-yl)methyl)-3-(trifluoromethyl)benzoyl chloride was used instead of benzoic chloride in Step 4 of Example 24. LCMS (ESI) : 671 (M + H)⁺.

### Example 43: N-(4-methyl-3-(7-methyl-2-((6-methylpyridin-3-yl)amino)-8-oxo-7,8-dihydropyrido[3,4-d]pyrimidin-6-yl)phenyl)methanesulfonamide

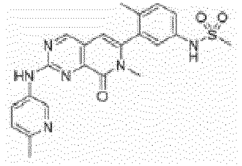

The experimental method was the same as that in Example 24, except that, in Step 4 of Example 24, methanesulfonic chloride was used instead of benzoic chloride and pyridine was used instead of diisopropylethylamine. LCMS (ESI): 451 (M + H)⁺**.**

### Example 44: N-(4-methyl-3-(7-methyl-2-((6-methylpyridin-3-yl)amino)-8-oxo-7,8-dihydropyrido[3,4-d]pyrimidin-6-yl)phenyl)-2-nitrobenzsulfonamide

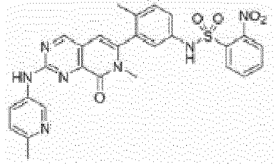

The experimental method was the same as that in Example 24, except that, in Step 4 of Example 24, 2-nitrobenzenesulfonyl chloride was used instead of benzoic chloride and pyridine was used instead of diisopropylethylamine. LCMS (ESI): 558 (M + H)⁺.

### Example 45: 3-bromo-N-(4-methyl-3-(7-methyl-2-((6-methylpyrimidin-3-yl)amino)-8-oxo-7,8-dihydropyrido[3,4-d]pyrimidin-6-yl)phenyl)benzenesulfonamide

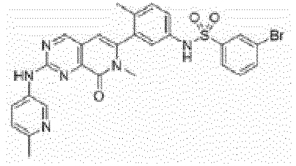

The experimental method was the same as that in Example 24, except that, in Step 4 of Example 24, 3-bromobenzenesulfonyl chloride was used instead of benzoic chloride and pyridine was used instead of diisopropylethylamine. LCMS (ESI): 591 (M + H)⁺.

### Example 46: 4-fluoro-N-(4-methyl-3-(7-methyl-2-((6-methylpyridin-3-yl)amino)-8-oxo-7,8-dihydropyrido[3,4-d]pyrimidin-6-yl)phenyl)benzenesulfonamide

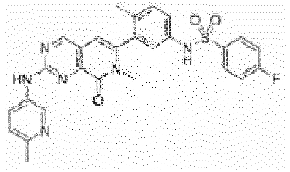

The experimental method was the same as that in Example 24, except that, in Step 4 of Example 24, 4-fluorbenzenesulfonyl chloride was used instead of benzoic chloride and pyridine was used instead of diisopropylethylamine. LCMS (ESI): 531 (M + H)⁺.

### Example 47: 1-cyclohexyl-3-(4-methyl-3-(7-methyl-2-((6-methylpyridin-3-yl)amino)-8-oxo-7,8-dihydropyrido[3,4-d]pyrimidin-6-yl)phenyl)urea

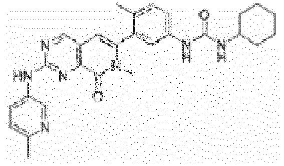

The experimental method was the same as that in Example 24, except that isocyanatocyclohexane was used instead of benzoic chloride in Step 4 of Example 24. LCMS (ESI): 498 (M + H)⁺.

### Example 48: 1-(2,3-dichlorophenyl)-3-(4-methyl-3-(7-methyl-2-((6-methylpyridin-3-yl)amino)-8-oxo-7,8-dihydropyrido[3,4-d]pyrimidin-6-yl) phenyl)urea

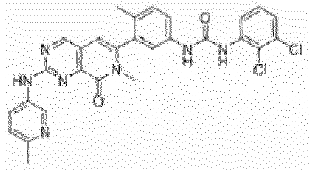

The experimental method was the same as that in Example 24, except that 1,2-dichloro-3-isocyanatobenzene was used instead of benzoic chloride in Step 4 of Example 24. LCMS (ESI): 560 (M + H)⁺.

### Example 49: 1-(2-methoxyphenyl)-3-(4-methyl-3-(7-methyl-2-((6-methylpyridin-3-yl)amino)-8-oxo-7,8-dihydropyrido[3,4-d]pyrimidin-6-yl)phenyl)urea

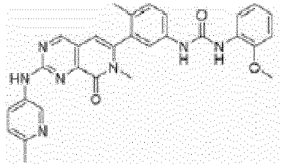

The experimental method was the same as that in Example 24, except that 1-isocyanato-2-methoxybenzene was used instead of benzoic chloride in Step 4 of Example 24. LCMS (ESI): 522 (M + H)⁺.

### Example 50: 6-(5-(ethylamino)-2-methylphenyl)-7-methyl-2-((6-methylpyridin-3-yl)amino)pyrido[3,4-d]pyridin-8(7H)-one

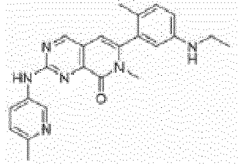

In a round bottom flask, 6-(5-amino-2-methylphenyl)-7-methyl-2-((6-methylpyridin-3-yl)amino)pyrido[3,4-d]pyrimidin-8 (7H)-one (50 mg, 1 eq.) produced in Step 3 of Example 24, acetic acid (1 drop) and acetaldehyde (3 eq.) were dissolved in 3 mL of dichloromethane. The reaction solution was stirred at room temperature for 10 minutes, and then sodium triacetoxyborohydride (85 mg, 3 eq.) was added thereto at 0°C, followed by stirring at room temperature for 6 hours. After completion of the reaction, the reaction solution was diluted with dichloromethane, and then washed with a saturated aqueous solution of ammonium chloride and then with brine. The organic layer was dried with magnesium sulfate and concentrated. The concentrate was purified by chromatography to obtain the title compound. LCMS (ESI): 401 (M + H)⁺.

### Example 51: 6-(5-((4-fluorobenzyl)amino)-2-methylphenyl)-7-methyl-2-((6-methylpyridin-3-yl)amino)pyrido[3,4-d]pyrimidin-8(7H)-one

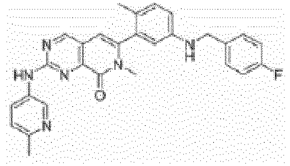

The experimental method was the same as that in Example 50, except that 4-fluorbenzenealdehyde was used instead of acetaldehyde. LCMS (ESI): 481 (M + H)⁺.

### Example 52: 6-(5-(((5-bromofuran-2-yl)methyl) amino)-2-methylphenyl)-7-methyl-2-((6-methylpyridin-3-yl)amino)pyrido[3,4-d]pyrimidin-8(7H)-one

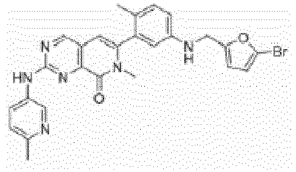

The experimental method was the same as that in Example 50, except that 5-bromofuran-2-carbalaldehyde was used instead of acetaldehyde. LCMS (ESI): 531 (M + H)⁺.

### Example 53: N-(3-(2-(cyclopropylamino)-8-oxo-7,8-dihydropyrido[3,4-d]pyrimidin-6-yl)-4-methylphenyl)-3-(trifluoromethyl)benzamide

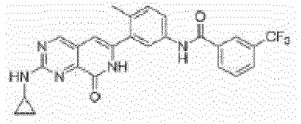

The compound and experimental method were the same as those in Example 1, except that methylation in Step 7 of Example 1 was not performed. LCMS (ESI): 480 (M + H)⁺.

### Example 54: N-(4-methyl-3-(2-((6-methylpyridin-3-yl)amino)-8-oxo-7,8-dihydropyrido[3,4-d]pyrimidine-6-yl)phenyl)-3-(trifluoromethyl)benzamide

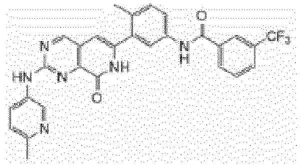

The compound and experimental method were the same as those in Example 1, except that methylation in Step 7 of Example 1 was not performed. LCMS (ESI): 531 (M + H)⁺.

### Example 55: N-(3-(2-amino-8-oxo-7,8-dihydropyrido[3,4-d]pyrimidin-6-yl)-4-methylphenyl)-3-(trifluoromethyl)benzamide

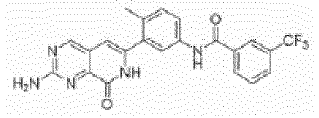

The compound and experimental method were the same as those in Example 53, except that an ammonia tetrahydrofuran solution was used instead of cyclopropylamine. LCMS (ESI): 440 (M + H)⁺.

### [Experimental Examples]

### Experimental Example 1. Measurement of Kinase Inhibitory Activity

In order to measure the protein kinase inhibitory activity (% inhibition) of the compound of the present disclosure, biochemical assay was performed in the full kinase panel shown in Table 1 below.

As a test compound, Compound No. 55 was used. The percent inhibition of each kinase when treated with the test compound at a single concentration of 1 µM was measured, and the residual enzyme activity values (%) of the kinases were calculated. Kinases whose calculated residual enzyme activity values (%) was 30% or less (i.e., kinases inhibited by 70% or more) are as follows:

### <Kinases inhibited by 70% or more>

ABL1, ABL2, ACK1, ARAF, BLK, BMX, BRK, c-Src, CSK, DDR1, DDR2, EPHA1, EPHA2, EPHA3, EPHA4, EPHA5, EPHA6, EPHA7, EPHA8, EPHB1, EPHB2, EPHB3, EPHB4, FGFR1, FGFR2, FGFR4, FLT1, FLT4, FMS, FRK, FYN, GCK, HCK, JAK1, JAK2, KDR, KHS, LCK, LYN, LYNB, p38a, p38b, PDGFRa, PDGFRb, PEAK1, RAF1, RET, RIPK3, SRMS, TAOK2, TIE2, TXK, TYK2, YES, YSK4, and ZAK.

### Experimental Example 2. Inhibitory Activities against GCK and ACK1 Kinases

The inhibitory activities of the compounds of the present disclosure against two kinases, GCK and ACK1, were measured, and the IC₅₀ values thereof were calculated. The calculated IC₅₀ values are shown in Table 1 below.

**[Table 1]**

| Test compound | Kinase inhibitory activity, IC₅₀ | |
|---|---|---|
| | ACK1 | GCK |
| Compound No. 13 | A | B |
| Compound No. 53 | A | B |
| Compound No. 55 | B | C |
| [Classification of IC₅₀] A: less than 0.1 µM, B: 0.1 to 1.0 µM, C: 1.0 µM to 10.0 µM, D: more than 10 µM | | |

### Experimental Example 3. Proliferation Inhibitory Activity

The inhibitory activities of the compounds of the present disclosure against the proliferation of the mt-NRAS (G12D) Ba/F3 and OCI-AML3 (mt-NRAS) cell lines were measured and the GI₅₀ values thereof were calculated. The calculated GI₅₀ values are shown in Table 2 below.

**[Table 2]**

| Test compound | Proliferation inhibitory activity (GI₅₀, µM) | |
|---|---|---|
| | OCI-AML3 (N-Ras Q61L) | Ba/F3 (N-Ras G12D) |
| Compound No. 1 | B | B |
| Compound No. 2 | B | B |
| Compound No. 6 | B | B |
| Compound No. 10 | D | D |
| Compound No. 11 | C | D |
| Compound No. 12 | B | D |
| Compound No. 13 | A | A |
| Compound No. 15 | B | B |
| Compound No. 17 | A | B |
| Compound No. 19 | B | B |
| Compound No. 22 | D | D |
| Compound No. 53 | A | A |
| Compound No. 54 | A | B |
| Compound No. 55 | A | A |
| [Classification of GI₅₀] A: less than 0.5 µM, B: 0.5 to 3.0 µM, C: 3.0 µM to 5.0 µM, D: more than 5.0 µM | | |

Referring to the results in Table 2, it can be seen that the compound of the present disclosure has inhibitory activity against the proliferation of the human acute myeloid leukemia cell line (OCI-AML3) having the NRAS mutant gene, and the effect thereof is remarkable. Therefore, it can be seen that the compound of the present disclosure is particularly effective as a therapeutic agent for acute myeloid leukemia (AML).

### [Formulation Examples]

Meanwhile, the novel compound represented by Formula 1 according to the present disclosure may be formulated in various forms depending on the intended use thereof. The following exemplifies several formulation methods containing, as an active ingredient, the compound represented by Formula 1 according to the present disclosure, but the scope of the present disclosure is not limited thereto.

### Formulation Example 1: Tablet (Direct Compression)

5.0 mg of the active ingredient was sieved and then mixed with 14.1 mg of lactose, 0.8 mg of crospovidone USNF and 0.1 mg of magnesium stearate, and the mixture was compressed into a tablet.

### Formulation Example 2: Tablet (Wet Granulation)

5.0 mg of the active ingredient was sieved and then mixed with 16.0 mg of lactose and 4.0 mg of starch. 0.3 mg of polysorbate 80 was dissolved in pure water, and then a suitable amount of the solution was added to the mixture, followed by atomization to obtain fine particles. After drying, the fine particles were sieved and then mixed with 2.7 mg of colloidal silicon dioxide and 2.0 mg of magnesium stearate. The fine particles were compressed into a tablet.

### Formulation Example 3: Powder and Capsule

5.0 mg of the active ingredient was sieved and then mixed with 14.8 mg of lactose, 10.0 g of polyvinylpyrrolidone and 0.2 mg of magnesium stearate. A No. 5 hard gelatin capsule was filled with the mixture using a suitable device.

### Formulation Example 4: Formulation Injection

A formulation for injection containing 100 mg of the active ingredient, 180 mg of mannitol, 26 mg of Na₂HPO₄·12H₂O and 2,974 mg of distilled water was prepared.

Although the embodiments of the present disclosure have been described above, those skilled in the art to which the present disclosure pertains will understand that the present disclosure may be embodied in other specific forms without departing from the technical spirit or essential features thereof. Therefore, it should be understood that the embodiments described above are illustrative in all respects, not restrictive.

## Claims

1. A compound selected from among a pyrido[3,4-d]pyrimidin-8-one derivative compound represented by the following Formula 1, a pharmaceutically acceptable salt thereof, a hydrate thereof, and a stereoisomer thereof: 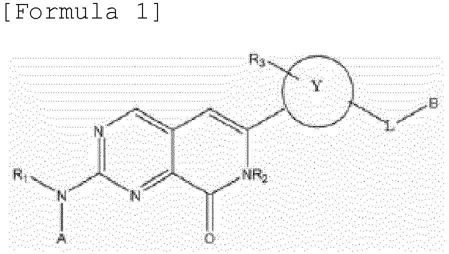 wherein
B is hydrogen, a C₁-C₁₃ alkyl group, a C₆-C₁₀ aryl group, a C₃-C₁₀ cyclyl group, a C₃-C₁₀ heteroaryl group, a C₃-C₁₀ heterocyclyl group, or -C (O)- (C₁-C₁₃ alkyl);
A is hydrogen, a C₁-C₁₃ alkyl group, a C₆-C₁₀ aryl group, a C₃-C₁₀ cyclyl group, a C₃-C₁₀ heteroaryl group, a C₃-C₁₀ heterocyclyl group, or -C (O)-(C₁-C₁₃ alkyl), or A together with a nitrogen atom to which R₁ is attached forms a 4- to 7-membered saturated, unsaturated or aromatic ring, which may optionally contain at least one of N, O, S, NH, C=N, C=O, -NHC(O)-, -NHC(O)NH-, -NHS(O)₂- and SO₂ and may optionally be substituted with at least one of a C₁-C₁₃ alkyl group, a C₆-C₁₀ aryl group, a C₃-C₁₀ heteroaryl group, a hydroxyl group, a halide group and a cyano group;
R₁ is hydrogen, a C₁-C₁₃ alkyl group, a C₃-C₁₀ cyclyl group, or a C₃-C₁₀ heterocyclyl group, or R₁ together with a nitrogen atom to which A is attached forms a 4- to 7-membered saturated, unsaturated or aromatic ring, which may optionally contain at least one of N, O, S, NH, C=N, C=O,-NHC(O)-, -NHC(O)NH-, -NHS(O)₂- and SO₂ and may optionally be substituted with at least one of a C₁-C₁₃ alkyl group, a C₆-C₁₀ aryl group, a C₃-C₁₀ heteroaryl group, a hydroxyl group, a halide group and a cyano group;
R₂ and R₃ are each hydrogen, a hydroxyl group, a halogen group, a C₁-C₆ alkyl group, a C₁-C₆ alkenyl group, a C₆-C₁₀ aryl group, a C₃-C₁₀ heteroaryl group, or a C₃-C₁₀ heterocyclyl group;
Y is a C₆-C₁₀ aryl group, or a 5- to 9-membered heteroaryl group containing 1 to 4 heteroatoms selected from among nitrogen (N), oxygen (O)and sulfur (S) atoms;
L is selected from the group consisting of -NR₄-,-NR₄CH₂-, -NR₄C(O)-, -C(O)NR₄-, -NR₄C(O)NR₄-, -S(O)₂-,-NR₄S(O)₂-, and -S (O)₂NR₄-;
R₄ is hydrogen or a C₁-C₆ alkyl group;
the C₁-C₆ alkyl group, the C₁-C₁₃ alkyl group or the C₃-C₁₀ cyclyl group contains at least one substituent selected from the group consisting of hydrogen, a hydroxyl group, a halogen group, a C₁-C₁₃ alkyl group, a C₁-C₆ alkoxy group, an amino group (-NR₅R₆), a nitro group (-N(O)₂), an amide group (-(C=O)NR₅R₆), a carboxylic acid group (-C(O)OH), a nitrile group (-CN), a urea group (-NR₅(C=O)NR₆-), a sulfonamide group (-NHS(O)₂-), a sulfide group (-S-), a sulfone group (-S(O)₂-), a phosphiryl group (-P(O)P₅P₆), a C₆-C₁₀ aryl group, a C₃-C₁₀ heteroaryl group, and a C₃-C₁₀ heterocyclyl group;
the C₆-C₁₀ aryl group, the C₃-C₁₀ heteroaryl group or the C₃-C₁₀ heterocyclyl group contains at least one substituent selected from the group consisting of hydrogen, a hydroxyl group, a halogen group, a carbonyl group (-(C=O)R₅R₆), a C₁-C₃ alkyl group unsubstituted or substituted with a halogen or C₃-C₁₀ heterocyclyl group, a C₁-C₃ alkoxy group unsubstituted or substituted with a halogen or C₃-C₁₀ heterocyclyl group, C₆-C₁₀ phenoxy, an amino group (-NR₅R₆), a nitro group (-N(O)₂), an amide group (-(C=O)NR₅R₆), a carboxylic acid group (-C(O)OH), a nitrile group (-CN), a urea group (-NR₅(C=O)NR₆-), a sulfonamide group (-NHS(O)₂-), a sulfide group (-S-), a sulfone group (-S(O)₂-), a phosphiryl group (-P(O)R₅R₆), a C₆-C₁₀ aryl group, a C₃-C₁₀ heteroaryl group, and a C₃-C₁₀ heterocyclyl group;
R₅ and R₆ contain at least one selected from the group consisting of hydrogen, a C₁-C₆ alkyl group, a C₁-C₆ alkenyl group, a C₁-C₆ alkynyl group, a C₆-C₁₀ aryl group, a C₃-C₁₀ heteroaryl group, and a C₃-C₁₀ heterocyclyl group; and
the C₃-C₁₀ heteroaryl group and the C₃-C₁₀ heterocyclyl group contain at least one heteroatom selected from the group consisting of N, O and S.

2. The compound of claim 1, wherein the pyrido[3,4-d]pyrimidin-8-one derivative compound represented by Formula 1 is any one of compounds represented by the following Formulas 2 to 9: 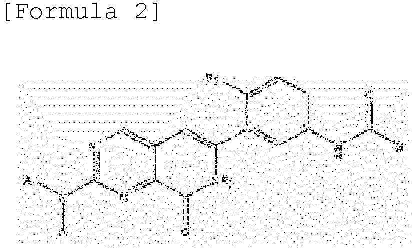 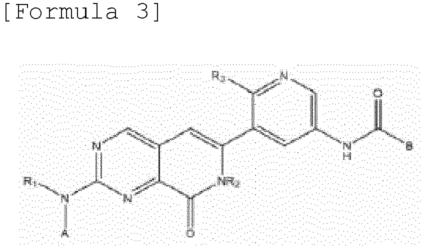 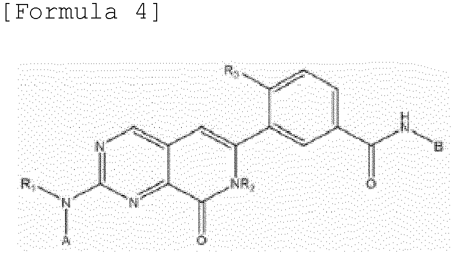 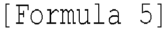 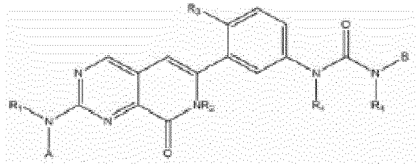 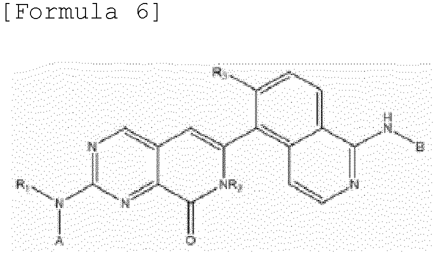 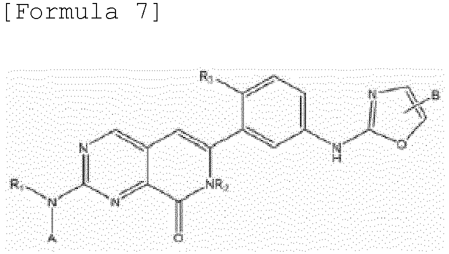 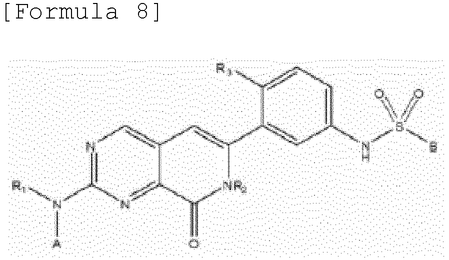 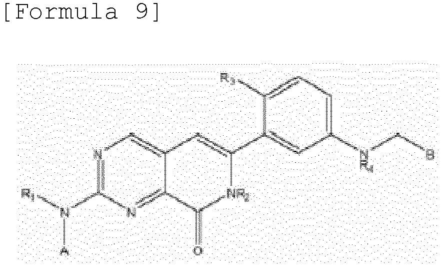 wherein
B is hydrogen, a C₁-C₁₃ alkyl group, a C₆-C₁₀ aryl group, a C₃-C₁₀ cyclyl group, a C₃-C₁₀ heteroaryl group, a C₃-C₁₀ heterocyclyl group, or -C(O)-(C₁-C₁₃ alkyl);
A is hydrogen, a C₁-C₁₃ alkyl group, a C₆-C₁₀ aryl group, a C₃-C₁₀ cyclyl group, a C₃-C₁₀ heteroaryl group, a C₃-C₁₀ heterocyclyl group, or -C(O)-(C₁-C₁₃ alkyl), or A together with a nitrogen atom to which R₁ is attached forms a 4- to 7-membered saturated, unsaturated or aromatic ring, which may optionally contain at least one of N, O, S, NH, C=N, C=O, -NHC(O)-, -NHC(O)NH-, -NHS(O)₂- and SO₂ and may optionally be substituted with at least one of a C₁-C₁₃ alkyl group, a C₆-C₁₀ aryl group, a C₃-C₁₀ heteroaryl group, a hydroxyl group, a halide group and a cyano group;
R₁ is hydrogen, a C₁-C₁₃ alkyl group, a C₃-C₁₀ cyclyl group, or a C₃-C₁₀ heterocyclyl group, or R₁ together with a nitrogen atom to which A is attached forms a 4- to 7-membered saturated, unsaturated or aromatic ring, which may optionally contain at least one of N, O, S, NH, C=N, C=O,-NHC(O)-, -NHC(O)NH-, -NHS(O)₂- and SO₂ and may optionally be substituted with at least one of a C₁-C₁₃ alkyl group, a C₆-C₁₀ aryl group, a C₃-C₁₀ heteroaryl group, a hydroxyl group, a halide group and a cyano group;
R₂ and R₃ are each hydrogen, a hydroxyl group, a halogen group, a C₁-C₆ alkyl group, a C₁-C₆ alkenyl group, a C₆-C₁₀ aryl group, a C₃-C₁₀ heteroaryl group, or a C₃-C₁₀ heterocyclyl group;
R₄ is hydrogen or a C₁-C₆ alkyl group;
the C₁-C₆ alkyl group, the C₁-C₁₃ alkyl group or the C₃-C₁₀ cyclyl group contains at least one substituent selected from the group consisting of hydrogen, a hydroxyl group, a halogen group, a C₁-C₁₃ alkyl group, a C₁-C₆ alkoxy group, an amino group (-NR₅R₆), a nitro group (-N(O)₂), an amide group (-(C=O)NR₅R₆), a carboxylic acid group (-C(O)OH), a nitrile group (-CN), a urea group (-NR₅(C=O)NR₆-), a sulfonamide group (-NHS(O)₂-), a sulfide group (-S-), a sulfone group (-S(O)₂-), a phosphiryl group (-P(O)P₅P₆), a C₆-C₁₀ aryl group, a C₃-C₁₀ heteroaryl group, and a C₃-C₁₀ heterocyclyl group;
the C₆-C₁₀ aryl group, the C₃-C₁₀ heteroaryl group or the C₃-C₁₀ heterocyclyl group contains at least one substituent selected from the group consisting of hydrogen, a hydroxyl group, a halogen group, a carbonyl group (-(C=O)R₅R₆), a C₁-C₃ alkyl group unsubstituted or substituted with a halogen or C₃-C₁₀ heterocyclyl group, a C₁-C₃ alkoxy group unsubstituted or substituted with a halogen or C₃-C₁₀ heterocyclyl group, C₆-C₁₀ phenoxy, an amino group (-NR₅R₆), a nitro group (-N(O)₂), an amide group (-(C=O)NR₅R₆), a carboxylic acid group (-C(O)OH), a nitrile group (-CN), a urea group (-NR₅(C=O)NR₆-), a sulfonamide group (-NHS(O)₂-), a sulfide group (-S-), a sulfone group (-S(O)₂-), a phosphiryl group (-P(O)R₅R₆), a C₆-C₁₀ aryl group, a C₃-C₁₀ heteroaryl group, and a C₃-C₁₀ heterocyclyl group;
R₅ and R₆ contain at least one selected from the group consisting of hydrogen, a C₁-C₆ alkyl group, a C₁-C₆ alkenyl group, a C₁-C₆ alkynyl group, a C₆-C₁₀ aryl group, a C₃-C₁₀ heteroaryl group, and a C₃-C₁₀ heterocyclyl group; and
the C₃-C₁₀ heteroaryl group and the C₃-C₁₀ heterocyclyl group contain at least one heteroatom selected from the group consisting of N, O and S.

3. The compound of claim 2, wherein
B is hydrogen, a C₁-C₁₃ alkyl group, a C₆-C₁₀ aryl group, a C₃-C₁₀ cyclyl group, or a C₃-C₁₀ heteroaryl group;
A is hydrogen, a C₁-C₁₃ alkyl group, a C₆-C₁₀ aryl group, a C₃-C₁₀ cyclyl group, or a C₃-C₁₀ heteroaryl group, or A together with a nitrogen atom to which R₁ is attached forms a 4- to 7-membered saturated or unsaturated ring, which contains at least one of N, O, S, NH, C=N, C=O,-NHC(O)-, -NHC(O)NH-, -NHS(O)₂- and SO₂ and may optionally be substituted with at least one of a C₁-C₁₃ alkyl group, a C₆-C₁₀ aryl group, a C₃-C₁₀ heteroaryl group, a hydroxyl group, a halide group and a cyano group;
R₁ is hydrogen or a C₁-C₁₃ alkyl group, or R₁ together with a nitrogen atom to which A is attached forms a 4- to 7-membered saturated or unsaturated ring, which contains at least one of N, O, S, NH, C=N, C=O, -NHC(O)-, -NHC(O)NH-,-NHS(O)₂- and SO₂ and may optionally be substituted with at least one of a C₁-C₁₃ alkyl group, a C₆-C₁₀ aryl group, a C₃-C₁₀ heteroaryl group, a hydroxyl group, a halide group and a cyano group;
R₂ and R₃ are each hydrogen, a halogen group, a C₁-C₆ alkyl group, or a C₁-C₆ alkenyl group;
the C₁-C₆ alkyl group, the C₁-C₁₃ alkyl group or the C₃-C₁₀ cyclyl group contains at least one substituent selected from the group consisting of hydrogen, a hydroxyl group, a halogen group, a C₁-C₁₃ alkyl group, a C₁-C₆ alkoxy group, an amide group (-(C=O)NR₅R₆), a C₆-C₁₀ aryl group, a C₃-C₁₀ heteroaryl group, and a C₃-C₁₀ heterocyclyl group;
the C₁-C₆ alkyl group, the C₁-C₁₃ alkyl group or the C₃-C₁₀ cyclyl group contains at least one substituent selected from the group consisting of hydrogen, a hydroxyl group, a halogen group, a C₁-C₁₃ alkyl group, a C₁-C₆ alkoxy group, an amino group (-NR₅R₆), a nitro group (-N(O)₂), an amide group (-(C=O)NR₅R₆), a carboxylic acid group (-C(O)OH), a nitrile group (-CN), a urea group (-NR₅(C=O)NR₆-), a sulfonamide group (-NHS(O)₂-), a sulfide group (-S-), a sulfone group (-S(O)₂-), a phosphiryl group (-P(O)P₅P₆), a C₆-C₁₀ aryl group, a C₃-C₁₀ heteroaryl group, and a C₃-C₁₀ heterocyclyl group;
the C₆-C₁₀ aryl group, the C₃-C₁₀ heteroaryl group or the C₃-C₁₀ heterocyclyl group contains at least one substituent selected from the group consisting of hydrogen, a hydroxyl group, a halogen group, a carbonyl group (-(C=O)R₅R₆), a C₁-C₃ alkyl group unsubstituted or substituted with a halogen or C₃-C₁₀ heterocyclyl group, a C₁-C₃ alkoxy group unsubstituted or substituted with a halogen or C₃-C₁₀ heterocyclyl group, C₆-C₁₀ phenoxy, an amino group (-NR₅R₆), a nitro group (-N(O)₂), an amide group (-(C=O)NR₅R₆), a carboxylic acid group (-C(O)OH), a nitrile group (-CN), a urea group (-NR₅(C=O)NR₆-), a sulfonamide group (-NHS(O)₂-), a sulfide group (-S-), a sulfone group (-S(O)₂-), a phosphiryl group (-P(O)R₅R₆), a C₆-C₁₀ aryl group, a C₃-C₁₀ heteroaryl group, and a C₃-C₁₀ heterocyclyl group;
R₅ and R₆ contain at least one selected from the group consisting of hydrogen, a C₁-C₆ alkyl group, a C₁-C₆ alkenyl group, a C₁-C₆ alkynyl group, a C₆-C₁₀ aryl group, a C₃-C₁₀ heteroaryl group, and a C₃-C₁₀ heterocyclyl group; and
the C₃-C₁₀ heteroaryl group and the C₃-C₁₀ heterocyclyl group contain at least one heteroatom selected from the group consisting of N, O and S.

4. The compound of claim 2, wherein
B is a C₁-C₁₃ alkyl group, a C₆-C₁₀ aryl group, a C₃-C₁₀ cyclyl group, or a C₃-C₁₀ heteroaryl group;
A is hydrogen, a C₁-C₁₃ alkyl group, a C₆-C₁₀ aryl group, a C₃-C₁₀ cyclyl group, or a C₃-C₁₀ heteroaryl group, or A together with a nitrogen atom to which R₁ is attached forms a 4- to 7-membered saturated or unsaturated ring containing at least one of N, O, S, NH, C=N, C=O, -NHC(O)-, -NHC(O)NH-, -NHS(O)₂- and SO₂;
R₁ is hydrogen, a C₁-C₁₃ alkyl group, or a C₃-C₁₀ cyclyl group, or R₁ together with a nitrogen atom to which A is attached forms a 4- to 7-membered saturated or unsaturated ring containing at least one of N, O, S, NH, C=N, C=O,-NHC(O)-, -NHC(O)NH-, -NHS(O)₂- and SO₂;
R₂ is hydrogen, a halogen group, a C₁-C₆ alkyl group, or a C₁-C₆ alkenyl group;
R₃ is a C₁-C₆ alkyl group;
R₄ is hydrogen or a C₁-C₆ alkyl group;
the C₁-C₆ alkyl group, the C₁-C₁₃ alkyl group or the C₃-C₁₀ cyclyl group contains at least one substituent selected from the group consisting of hydrogen, a hydroxyl group, a halogen group, a C₁-C₁₃ alkyl group, a C₁-C₆ alkoxy group, an amino group (-NR₅R₆), a nitro group (-N(O)₂), an amide group (-(C=O)NR₅R₆), a carboxylic acid group (-C(O)OH), a nitrile group (-CN), a urea group (-NR₅(C=O)NR₆-), a sulfonamide group (-NHS(O)₂-), a sulfide group (-S-), a sulfone group (-S(O)₂-), a phosphiryl group (-P(O)R₅R₆), a C₆-C₁₀ aryl group, a C₃-C₁₀ heteroaryl group, and a C₃-C₁₀ heterocyclyl group;
the C₆-C₁₀ aryl group, the C₃-C₁₀ heteroaryl group or the C₃-C₁₀ heterocyclyl group contains at least one substituent selected from the group consisting of hydrogen, a hydroxyl group, a halogen group, a carbonyl group (-(C=O)R₅R₆), a C₁-C₃ alkyl group unsubstituted or substituted with a halogen or C₃-C₁₀ heterocyclyl group, a C₁-C₃ alkoxy group unsubstituted or substituted with a halogen or C₃-C₁₀ heterocyclyl group, C₆-C₁₀ phenoxy, an amino group (-NR₅R₆), a nitro group (-N(O)₂), an amide group (-(C=O)NR₅R₆), a carboxylic acid group (-C(O)OH), a nitrile group (-CN), a urea group (-NR₅(C=O)NR₆-), a sulfonamide group (-NHS(O)₂-), a sulfide group (-S-), a sulfone group (-S(O)₂-), a phosphiryl group (-P(O)R₅R₆), a C₆-C₁₀ aryl group, a C₃-C₁₀ heteroaryl group, and a C₃-C₁₀ heterocyclyl group;
R₅ and R₆ contain at least one selected from the group consisting of hydrogen, a C₁-C₆ alkyl group, a C₁-C₆ alkenyl group, a C₁-C₆ alkynyl group, a C₆-C₁₀ aryl group, a C₃-C₁₀ heteroaryl group, and a C₃-C₁₀ heterocyclyl group; and
the C₃-C₁₀ heteroaryl group and the C₃-C₁₀ heterocyclyl group contain at least one heteroatom selected from the group consisting of N, O and S.

5. The compound of claim 2, wherein
B is a C₁-C₆ alkyl group, substituted or unsubstituted phenyl, substituted or unsubstituted hexane, substituted or unsubstituted furan, substituted or unsubstituted thiophene, substituted or unsubstituted pyridine, substituted or unsubstituted benzofuran, substituted or unsubstituted benzene, substituted or unsubstituted naphthalene, substituted or unsubstituted anthracene, or substituted or unsubstituted phenanthrene;
A is hydrogen, substituted or unsubstituted pyridazine, substituted or unsubstituted pyrazine; substituted or unsubstituted imidazole, substituted or unsubstituted pyrazole, substituted or unsubstituted furan, substituted or unsubstituted pyrimidine, substituted or unsubstituted pyrrole, substituted or unsubstituted pyridine, substituted or unsubstituted cyclopropane, substituted or unsubstituted cyclobutane, substituted or unsubstituted ethane, substituted or unsubstituted butane, or substituted or unsubstituted pentane, or A together with a nitrogen atom to which R₁ is attached forms a substituted or unsubstituted morpholino group;
R₁ is hydrogen, or R₁ together with a nitrogen atom to which A is attached forms a substituted or unsubstituted morpholino group;
R₂ is hydrogen or a substituted or unsubstituted C₁-C₃ alkyl group;
R₃ is hydrogen or a C₁-C₃ alkyl group; and
R₄ is hydrogen or a C₁-C₆ alkyl group.

6. The compound of claim 1, wherein the compound is any one selected from the group consisting of the following Compound Nos. 1 to 55:
(Compound No. 1) N-(3-(2-(cyclopropylamino)-7-methyl-8-oxo-7,8-dihydropyrido[3,4-d]pyrimidin-6-yl)-4-methylphenyl)-3-(trifluoromethyl)benzamide;
(Compound No. 2) N-(3-(2-((2-hydroxyethyl)amino)-7-methyl-8-oxo-7,8-dihydropyrido[3,4-d]pyrimidin-6-yl)-4-methylphenyl)-3-(trifluoromethyl)benzamide;
(Compound No. 3) N-(4-methyl-3-(7-methyl-2-(methylamino)-8-oxo-7,8-dihydropyrido[3,4-d]pyrimidin-6-yl)phenyl)-3-(trifluoromethyl)benzamide;
(Compound No. 4) N-(4-methyl-3-(7-methyl-2-(oxetan-3-ylamino)-8-oxo-7,8-dihydropyrido[3,4-d]pyrimidin)-6-yl)phenyl)-3-(trifluoromethyl)benzamide;
(Compound No. 5) N-(4-methyl-3-(7-methyl-2-((oxetan-2-ylmethyl)amino)-8-oxo-7,8-dihydropyrido[3,4-d]pyrimidin-6-yl)phenyl)-3-(trifluoromethyl)benzamide;
(Compound No. 6) N-(4-methyl-3-(7-methyl-2-((2-morpholinoethyl)amino)-8-oxo-7,8-dihydropyrido[3,4-d]pyrimidin-6-yl)phenyl)-3-(trifluoromethyl)benzamide;
(Compound No. 7) N-(4-methyl-3-(7-methyl-8-oxo-2-((2-(thiazol-2-yl) ethyl)amino)-7,8-dihydropyrido[3,4-d]pyrimidin-6-yl)phenyl)-3-(trifluoromethyl)benzamide;
(Compound No. 8) N-(4-methyl-3-(7-methyl-8-oxo-2-((2-(thiophen-2-yl) ethyl)amino)-7,8-dihydropyrido[3,4-d]pyrimidin-6-yl)phenyl)-3-(trifluoromethyl)benzamide;
(Compound No. 9) N-(4-methyl-3-(7-methyl-2-((2-(4-nitrophenoxy)ethyl)amino)-8-oxo-7,8-dihydropyrido[3,4-d]pyrimidin-6-yl)phenyl)-3-(trifluoromethyl)benzamide;
(Compound No. 10) N-(3-(2-((4-methoxybenzyl)amino)-7-methyl-8-oxo-7,8-dihydropyrido[3,4-d]pyrimidin-6-yl)-4-methylphenyl)-3-(trifluoromethyl)benzamide;
(Compound No. 11) N-(3-(2-((2-((furan-2-ylmethyl)thio)ethyl)amino)-7-methyl-8-oxo-7,8-dihydiropyrido[3,4-d]pyrimidin-6-yl)-4-methylphenyl)-3-(trifluoromethyl)benzamide;
(Compound No. 12) N-(4-methyl-3-(7-methyl-2-morpholino-8-oxo-7,8-dihydropyrido[3,4-d]pyrimidin-6-yl)phenyl)-3-(trifluoromethyl)benzamide;
(Compound No. 13) N-(4-methyl-3-(7-methyl-2-((6-methylpyridin-3-yl)amino)-8-oxo-7,8-dihydropyrido[3,4-d]pyrimidin-6-yl)phenyl)-3-(trifluoromethyl)benzamide;
(Compound No. 14) N-(4-methyl-3-(7-methyl-8-oxo-2-(phenylamino)-7,8-dihydropyrido[3,4-d]pyrimidin-6-yl)phenyl)-3-(trifluoromethyl)benzamide;
(Compound No. 15) N,N-dimethyl-4-((7-methyl-6-(2-methyl-5-(3-(trifluoromethyl)benzamido)phenyl)-8-oxo-7,8-dihydropyrimido[3,4-d]pyrimidin-2-yl)amino)benzamide;
(Compound No. 16) N-(4-methyl-3-(7-methyl-2-((1-methyl-1H-pyrazol-4-yl)amino)-8-oxo-7,8-dihydropyrido[3,4-d]pyrimidin-6-yl)phenyl)-3-(trifluoromethyl)benzamide;
(Compound No. 17) N-(3-(2-((4-(4-ethylpiperazin-1-yl)phenyl)amino)-7-methyl-8-oxo-7,8-dihydropyrido[3,4-d]pyrimidin-6-yl)-4-methylphenyl)-3-(trifluoromethyl)benzamide;
(Compound No. 18) N-(3-(2-((3-(4-ethylpiperazin-1-yl)phenyl)amino)-7-methyl-8-oxo-7,8-dihydropyrido[3,4-d]pyrimidin-6-yl)-4-methylphenyl)-3-(trifluoromethyl)benzamide;
(Compound No. 19) N-(3-(2-((4-(4-hydroxypiperidin-1-yl)phenyl)amino)-7-methyl-8-oxo-7,8-dihydropyrido[3,4-d]pyrimidin-6-yl)-4-methylphenyl)-3-(trifluoromethyl)benzamide;
(Compound No. 20) N-(4-methyl-3-(7-methyl-2-((6-morpholinopyridin-3-yl)amino)-8-oxo-7,8-dihydropyrido[3,4-d]pyrimidin-6-yl)phenyl)-3-(trifluoromethyl)benzamide;
(Compound No. 21) N-(4-methyl-3-(7-methyl-2-((6-(4-(4-methylpiperazin-1-yl)piperidin-1-yl)pyridin3-yl)amino)-8-oxo-7,8-dihydropyrido[3,4-d]pyrimidin-6-yl)phenyl)-3-(trifluoromethyl)benzamide;
(Compound No. 22) N-(3-(2-((2-methoxy-4-morpholinophenyl)amino)-7-methyl-8-oxo-7,8-dihydropyrido[3,4-d]pyrimidin-6-yl)-4-methylphenyl)-3-(trifluoromethyl)benzamide;
(Compound No. 23) N-(4-methyl-3-(7-methyl-2-((4-((4-methylpiperazin-1-yl)methyl)-3-(trifluoromethyl)phenyl)amino)-8-oxo-7,8-dihydropyrido[3,4-d]pyrimidin-6-yl)phenyl)-3-(trifluoromethyl)benzamide;
(Compound No. 24) N-(4-methyl-3-(7-methyl-2-((6-methylpyridin-3-yl)amino)-8-oxo-7,8-dihydropyrido[3,4-d]pyrimidin-6-yl)phenyl)benzamide;
(Compound No. 25) 5-methyl-N-(4-methyl-3-(7-methyl-2-((6-methylpyridin-3-yl)amino)-8-oxo-7,8-dihydropyrido[3,4-d]pyrimidin-6-yl)phenyl)nicotinamide;
(Compound No. 26) N-(4-methyl-3-(7-methyl-2-((6-methylpyridin-3-yl)amino)-8-oxo-7,8-dihydiropyrido[3,4-d]pyrimidin-6-yl)phenyl)thiophene-3-carboxamide;
(Compound No. 27) N-(4-methyl-3-(7-methyl-2-((6-methylpyridin-3-yl)amino)-8-oxo-7,8-dihydropyrido[3,4-d]pyrimidin-6-yl)phenyl)benzofuran-2-carboxamide;
(Compound No. 28) N-(4-methyl-3-(7-methyl-2-((6-methylpyridin-3-yl)amino)-8-oxo-7,8-dihydropyrido[3,4-d]pyrimidin-6-yl)phenyl)-2-naphthamide;
(Compound No. 29) N-(4-methyl-3-(7-methyl-2-((6-methylpyridin-3-yl)amino)-8-oxo-7,8-dihydropyrido[3,4-d]pyrimidin-6-yl)phenyl)-2,3-dihydrobenzo[b] [1,4]dioxy-6-carboxamide;
(Compound No. 30) N-(4-methyl-3-(7-methyl-2-((6-methylpyridin-3-yl)amino)-8-oxo-7,8-dihydropyrido[3,4-d]pyrimidin-6-yl)phenyl)-2-(3-(trifluoromethyl)phenyl)acetamide;
(Compound No. 31) N-(4-methyl-3-(7-methyl-2-((6-methylpyridin-3-yl)amino)-8-oxo-7,8-dihydropyrido[3,4-d]pyrimidin-6-yl)phenyl)acetamide;
(Compound No. 32) N-(4-methyl-3-(7-methyl-2-((6-methylpyridin-3-yl)amino)-8-oxo-7,8-dihydropyrido[3,4-d]pyrimidin-6-yl)phenyl)-4-(4-methylpyperazin-1-yl)benzamide;
(Compound No. 33) N-(4-methyl-3-(7-methyl-2-((6-methylpyridin-3-yl)amino)-8-oxo-7,8-dihydropyrido[3,4-d]pyrimidin-6-yl)phenyl)propionamide;
(Compound No. 34) N-(4-methyl-3-(7-methyl-2-((6-methylpyridin-3-yl)amino)-8-oxo-7,8-dihydropyrido[3,4-d]pyrimidin-6-yl)phenyl)-4-morpholinobenzamide;
(Compound No. 35) N-(4-methyl-3-(7-methyl-2-((6-methylpyridin-3-yl)amino)-8-oxo-7,8-dihydropyrido[3,4-d]pyrimidin-6-yl)phenyl)-4-((4-methylpiperazin-1-yl)methyl)benzamide;
(Compound No. 36) N-(4-methyl-3-(7-methyl-2-((6-methylpyridin-3-yl)amino)-8-oxo-7,8-dihydropyrido[3,4-d]pyrimidin-6-yl)phenyl)-3-(4-methylpiperazin-1-yl)-5-(trifluoromethyl)benzamide;
(Compound No. 37) 3-(2,4-dimethyl-1H-imidazol-1-yl)-N-(4-methyl-3-(7-methyl-2-((6-methylpyridin-3-yl))amino)-8-oxo-7,8-dihydropyrido[3,4-d]pyrimidin-6-yl)phenyl)-5-(trifluoromethyl)benzamide;
(Compound No. 38) 3-(4-hydroxypiperidin-1-yl)-N-(4-methyl-3-(7-methyl-2-((6-methylpiperidin-3-yl)amino)-8-oxo-7,8-dihydropyrido[3,4-d]pyrimidin-6-yl)phenyl)-5-(trifluoromethyl)benzamide;
(Compound No. 39) 4-(4-methyl-1H-imidazol-1-yl)-N-(4-methyl-3-(7-methyl-2-((6-methylpyridin-3-yl)amino-8)-oxo-7,8-dihydropyrido[3,4-d]pyrimidin-6-yl)phenyl)-3-(trifluoromethyl)benzamide;
(Compound No. 40) N-(4-methyl-3-(7-methyl-2-((6-methylpyridin-3-yl)amino)-8-oxo-7,8-dihydropyrido[3,4-d]pyrimidin-6-yl)phenyl)-4-(4-methylpiperazin-1-yl)-3-(trifluoromethyl)benzamide;
(Compound No. 41) N-(4-methyl-3-(7-methyl-2-((6-methylpyridin-3-yl)amino)-8-oxo-7,8-dihydropyrido[3,4-d]pyrimidin-6-yl)phenyl)-4-(morpholinomethyl)-3-(trifluoromethyl)benzamide;
(Compound No. 42) 4-((3-(dimethylamino)pinolidin-1-yl)methyl)-N-(4-methyl-3-(7-methyl-2-((6-methylpyridine-3-yl)amino)-8-oxo-7,8-dihydropyrido[3,4-d]pyrimidin-6-yl)phenyl)-3-(trifluoromethyl)benzamide;
(Compound No. 43) N-(4-methyl-3-(7-methyl-2-((6-methylpyridin-3-yl)amino)-8-oxo-7,8-dihydropyrido[3,4-d]pyrimidin-6-yl)phenyl)methanesulfonamide;
(Compound No. 44) N-(4-methyl-3-(7-methyl-2-((6-methylpyridin-3-yl)amino)-8-oxo-7,8-dihydropyrido[3,4-d]pyrimidin-6-yl)phenyl)-2-nitrobenzsulfonamide;
(Compound No. 45) 3-bromo-N-(4-methyl-3-(7-methyl-2-((6-methylpyrimidin-3-yl)amino)-8-oxo-7,8-dihydropyrido[3,4-d]pyrimidin-6-yl)phenyl)benzenesulfonamide;
(Compound No. 46) 4-fluoro-N-(4-methyl-3-(7-methyl-2-((6-methylpyridin-3-yl)amino)-8-oxo-7,8-dihydropyrido[3,4-d]pyrimidin-6-yl)phenyl)benzenesulfonamide;
(Compound No. 47) 1-cyclohexyl-3-(4-methyl-3-(7-methyl-2-((6-methylpyridin-3-yl)amino)-8-oxo-7,8-dihydropyrido[3,4-d]pyrimidin-6-yl)phenyl)urea;
(Compound No. 48) 1-(2,3-dichlorophenyl)-3-(4-methyl-3-(7-methyl-2-((6-methylpyridin-3-yl)amino)-8-oxo-7,8-dihydropyrido[3,4-d]pyrimidin-6-yl)phenyl)urea;
(Compound No. 49) 1-(2-methoxyphenyl)-3-(4-methyl-3-(7-methyl-2-((6-methylpyridin-3-yl)amino)-8-oxo-7,8-dihydropyrido[3,4-d]pyrimidin-6-yl)phenyl)urea;
(Compound No. 50) 6-(5-(ethylamino)-2-methylphenyl)-7-methyl-2-((6-methylpyridin-3-yl)amino)pyrido[3,4-d]pyridin-8(7H)-one;
(Compound No. 51) 6-(5-((4-fluorobenzyl)amino)-2-methylphenyl)-7-methyl-2-((6-methylpyridin-3-yl)amino)pyrido[3,4-d]pyrimidin-8(7H)-one;
(Compound No. 52) 6-(5-(((5-bromofuran-2-yl)methyl)amino)-2-methylphenyl)-7-methyl-2-((6-methylpyridin-3-yl)amino)pyrido[3,4-d]pyrimidin-8(7H)-one;
(Compound No. 53) N-(3-(2-(cyclopropylamino)-8-oxo-7,8-dihydropyrido[3,4-d]pyrimidin-6-yl)-4-methylphenyl)-3-(trifluoromethyl)benzamide;
(Compound No. 54) N-(4-methyl-3-(2-((6-methylpyridin-3-yl)amino)-8-oxo-7,8-dihydropyrido[3,4-d]pyrimidin-6-yl)phenyl)-3-(trifluoromethyl)benzamide; and
(Compound No. 55) N-(3-(2-amino-8-oxo-7,8-dihydropyrido[3,4-d]pyrimidin-6-yl)-4-methylphenyl)-3-(trifluoromethyl)benzamide.

7. The compound of claim 1, wherein the pharmaceutically acceptable salt is a salt of an inorganic acid or organic acid selected from the group consisting of hydrochloric acid, hydrobromic acid, sulfuric acid, phosphoric acid, nitric acid, acetic acid, glycolic acid, lactic acid, pyruvic acid, malonic acid, succinic acid, glutaric acid, fumaric acid, malic acid, mandelic acid, tartaric acid, citric acid, ascorbic acid, palmitic acid, maleic acid, hydroxymaleic acid, benzoic acid, hydroxybenzoic acid, phenylacetic acid, cinnamic acid, salicylic acid, methanesulfonic acid, benzenesulfonic acid, and toluenesulfonic acid.

8. A pharmaceutical composition for preventing, alleviating or treating cancer containing the compound of any one of claims 1 to 7 as an active ingredient.

9. The pharmaceutical composition of claim 8, wherein the cancer is caused by NRAS mutation.

10. The pharmaceutical composition of claim 8, which is applied to a patient with NRAS mutation.

11. The pharmaceutical composition of claim 8, wherein the cancer is at least one selected from the group consisting of melanoma, colorectal cancer, thyroid cancer, and blood cancer.

12. The pharmaceutical composition of claim 8, wherein the cancer is acute myeloid leukemia (AML).

13. The pharmaceutical composition of claim 8, which is administered to a patient with NRAS G12D.
